# EUROPÄISCHE PATENTSCHRIFT

(11) **EP 3 011 182 B1**
(45) Veröffentlichungstag und Bekanntmachung des Hinweises auf die Patenterteilung: **30.09.2020**
(21) Anmeldenummer: 14735466.6
(22) Anmeldetag: 20.06.2014
(51) Int. Cl.: F04D 3/00, F04D 13/06, F04D 29/02, F04D 29/52, B22F 7/00

(54) **PUMPENGEHÄUSE AUS MINDESTENS ZWEI UNTERSCHIEDLICHEN VERSINTERBAREN MATERIALIEN**
PUMP HOUSING MADE FROM AT LEAST TWO DIFFERENT SINTERABLE MATERIALS
CORPS DE POMPE CONSTITUÉ D'AU MOINS DEUX MATÉRIAUX FRITTABLES DIFFÉRENTS

(30) Priorität: 21.06.2013 DE 102013211848
(43) Veröffentlichungstag der Anmeldung: 27.04.2016
(73) Patentinhaber: Heraeus Deutschland GmbH & Co. KG, 63450 Hanau (DE)
(72) Erfinder: SCHIBLI, Stefan, 60326 Frankfurt (DE); HAUSCH, Ulrich, 60318 Frankfurt (DE)
(74) Vertreter: Herzog IP Patentanwalts GmbH
(86) Internationale Anmeldenummer: PCT/EP2014/001684
(87) Internationale Veröffentlichungsnummer: WO 2014/202225

(56) Entgegenhaltungen:
- EP-A1- 2 236 229
- DE-U1- 29 723 409
- US-A- 4 603 062

## Beschreibung

Die Erfindung betrifft eine Pumpvorrichtung, beinhaltend i. einen Impeller; ii. ein Pumpengehäuse, das einen Innenbereich zumindest zu einem Teil umgibt, mit einem Einlass und einem Auslass, wobei der Impeller im Innenbereich des Pumpengehäuses vorgesehen ist; wobei das Pumpengehäuse mindestens einen ersten Teilbereich und mindestens einen weiteren Teilbereich beinhaltet; wobei der erste Teilbereich eine Keramik beinhaltet, wobei der weitere Teilbereich ein Metall beinhaltet, wobei mindestens ein Teil des ersten Teilbereiches und mindestens ein Teil des weiteren Teilbereiches miteinander verbunden sind. Weiterhin betrifft die Erfindung ein Gehäuse, das die für das Pumpengehäuse beschriebenen Merkmale beinhaltet.

Pumpvorrichtungen mit Rotoren oder Impellern sind bekannt. Manche Pumpvorrichtungen weisen als Förderstrecke für ein zu förderndes Fluid ein Pumpengehäuse in Form eines Rohres auf. Darin befindet sich oftmals ein Impeller, der zum Beispiel von einem außerhalb der Förderstrecke gelegenen Motor über eine Antriebswelle angetrieben wird. Das Pumpengehäuse ist über ein oder mehrere Halteelemente an der Pumpvorrichtung befestigt. Diese Art der Halterung kann verschiedene Nachteile beinhalten. Zum einen wird ein zusätzlicher Arbeitsschritt zum Anbringen der Halterung benötigt. Das erhöht die Herstellungskosten und ist ressourcenineffizient. Weiterhin ist die Verbindung zwischen dem Pumpengehäuse und der Halterung herstellungsbedingt oder aufgrund der eingesetzten Verbindungsmittel, z.B. Schrauben oder Nieten, nicht ohne Spannung. Dies liegt daran, dass für die Halterungen und/oder Verbindungsmittel meist andere Materialien ausgewählt werden als für das Pumpengehäuse. Durch diese Spannungen verschlechtern sich die Verbindungen der Halterung mit dem Pumpengehäuse mit der Zeit. Darüber hinaus ist es vor allem für sehr kleine Pumpen äußerst wichtig platzsparend hergestellt zu werden. Dies gilt insbesondere für Pumpen, die in einen Körper implantiert werden sollen. Eine platzsparende Konstruktion ist für Pumpen mit einer Vielzahl von Einzelteilen schwerer realisierbar als bei einer Pumpe mit einer kleineren Anzahl von Einzelteilen.

EP 2 236 229 A1 beschreibt ein Verfahren zum Herstellen eines Pumpengehäuses durch Sintern einer Form beinhaltend unterschiedliche Pulver.

Allgemein liegt eine Aufgabe der vorliegenden Erfindung darin, die sich aus dem Stand der Technik ergebenden Nachteile zumindest teilweise zu überwinden.

Eine weitere Aufgabe besteht darin, eine Pumpvorrichtung bereitzustellen deren Materialien möglichst biokompatibel, leicht verarbeitbar, korrosionsbeständig und dauerhaft miteinander verbindbar sind.

Eine weitere Aufgabe besteht darin, eine Pumpvorrichtung bereitzustellen, die möglichst platzsparend ausgestaltet ist.

Weiterhin besteht eine Aufgabe darin, eine möglichst spannungsfreie Pumpvorrichtung bereitzustellen, insbesondere mit einem möglichst spannungsfreien Gehäuse bzw. Pumpengehäuse, und insbesondere einen möglichst spannungsfreien Übergang vom Pumpengehäuse zum restlichen Teil der Pumpvorrichtung bereitzustellen.

Es besteht zudem eine Aufgabe darin, eine Pumpvorrichtung bereitzustellen, die einen möglichst geringen Abrieb der beweglichen Teile und deren Halterungen bei Benutzung aufweist.

Darüber hinaus besteht eine Aufgabe darin, ein Pumpengehäuse für eine Pumpvorrichtung bereitzustellen, die einfach und platzsparend in andere Bauteile, z.B. ein Bauteilgehäuse der Pumpvorrichtung integrierbar ist.

Darüber hinaus besteht eine Aufgabe darin, ein Pumpengehäuse für eine Pumpvorrichtung bereitzustellen, das hermetisch dicht mit einem Bauteilgehäuse der Pumpvorrichtung verbunden werden kann.

Weiterhin besteht eine Aufgabe darin, ein Gehäuse oder Pumpengehäuse bereitzustellen, das möglichst frei von inneren und / oder äußeren Spannungen ist.

Weiterhin besteht eine Aufgabe darin, ein Verfahren bereitzustellen, um ein Pumpengehäuse möglichst kosten- und zeitsparend herstellen zu können.

Es ist weiterhin eine Aufgabe, ein Bauteilgehäuse bereitzustellen, das möglichst platzsparend ausgestaltet ist.

Eine weitere Aufgabe ist ein Gehäuse bereitzustellen, das hermetisch dicht mit anderen Bauteilen verbunden werden kann.

Ein erster Gegenstand der vorliegenden Erfindung ist eine Pumpvorrichtung, beinhaltend:
i. einen Impeller;
ii. ein Pumpengehäuse, das einen Innenbereich zumindest zu einem Teil umgibt, mit einem Einlass und einem Auslass,
   wobei der Impeller im Innenbereich des Pumpengehäuses vorgesehen ist;
   wobei das Pumpengehäuse mindestens einen ersten Teilbereich und mindestens einen weiteren Teilbereich beinhaltet;
   wobei der erste Teilbereich zu mindestens 61 Gew.-%, bevorzugt zu mindestens 70 Gew.-%, oder bevorzugt zu mindestens 90 Gew.-%, bezogen auf die Gesamtmasse des ersten Teilbereiches, eine Keramik beinhaltet,
   wobei der weitere Teilbereich einen Metallgehalt in einem Bereich von 40 bis 90 Gew.-%, bezogen auf die Gesamtmasse des weiteren Teilbereiches, ein Metall beinhaltet,
   wobei mindestens ein Teil des ersten Teilbereiches und mindestens ein Teil des weiteren Teilbereiches miteinander verbunden sind.

Die erfindungsgemäße Pumpvorrichtung ist bevorzugt dazu geeignet in den Körper eines Menschen oder eines Tieres eingebracht zu werden. Die erfindungsgemäße Pumpvorrichtung ist ferner bevorzugt dazu ausgelegt, Körperflüssigkeiten wie Blut, Serum, Plasma, Interstitielle Flüssigkeit, Speichel oder Urin zu fördern. Insbesondere ist es bevorzugt, die erfindungsgemäße Pumpvorrichtung zum Fördern von Blut in den Blutkreislauf eines Menschen oder Tieres einzubringen. Das Einbringen der erfindungsgemäßen Pumpvorrichtung kann beispielsweise ein Implantieren in den Körper, ein Aufsetzen auf den Körper oder ein Verbinden mit dem Körper beinhalten.

Das Pumpengehäuse der erfindungsgemäßen Pumpvorrichtung kann jede Form aufweisen, die der Fachmann für den Einsatz in einer Pumpvorrichtung auswählen würde. Das Pumpengehäuse weist bevorzugt mindestens eine Wand des Pumpengehäuses, im Folgenden auch als Pumpengehäusewand bezeichnet, auf. Die mindestens eine Wand des Pumpengehäuses umgibt den Innenbereich des Pumpengehäuses. Das Pumpengehäuse weist mindestens zwei Enden auf, wobei mindestens ein Einlass an dem einen Ende und mindestens ein Auslass an dem anderen Ende angeordnet sind. Der Innenbereich des Pumpengehäuses ist, außer an dem Einlass und Auslass des Pumpengehäuses, von der Wand vollständig umgeben. Das Pumpengehäuse kann sich zum Teil über den Innenbereich des Pumpengehäuses hinaus erstrecken. Bevorzugt endet das Pumpengehäuse an dem Einlass bzw. Auslass.

Die dem Innenbereich abgewandte Seite des Pumpengehäuses wird als Außenseite des Pumpengehäuses bezeichnet. Das Pumpengehäuse weist bevorzugt eine längliche Form auf. Das Pumpengehäuse wird in seiner Form durch eine Längsausdehnung und mindestens einen Querschnitt definiert. Ein Querschnitt des Pumpengehäuses wird immer in einer Ebene bestimmt, die senkrecht zu der Pumpengehäusewand steht. Ist die Pumpengehäusewand in der Längsausdehnung gekrümmt, so wird ein Querschnitt senkrecht zur Tangente an einem Punkt auf der Pumpengehäusewand ermittelt. Als Längsausdehnung wird die Ausdehnung des Pumpengehäuses in Pumprichtung angesehen. Es gilt stets die kürzeste, gedachte Verbindung von Einlass und Auslass innerhalb des Pumpengehäuses. Die Pumpengehäusewand, auch als Wand bezeichnet, erstreckt sich in Richtung der Längsausdehnung des Pumpengehäuses. Die mindestens eine Wand kann eine oder mehrere Wandflächen aufweisen. Weist das Pumpengehäuse mehr als eine Wandfläche auf, sind diese über Ecken, an denen die Wandflächen zusammenlaufen, miteinander verbunden. Die Wand, sowie bevorzugt auch die Wandflächen, des Pumpengehäuses verlaufen bevorzugt parallel zur Längsausdehnung des Pumpengehäuses. Ein Teil der Pumpengehäusewand kann sich über den Innenbereich des Pumpengehäuses hinaus erstrecken. Bevorzugt erstreckt sich die Pumpengehäusewand über den gesamten Innenbereich des Pumpengehäuses. Ist das Pumpengehäuse röhrenförmig ausgestaltet, befinden sich der Einlass an dem ersten Ende und der Auslass an dem gegenüberliegenden Ende des Pumpengehäuses. An den Enden des Pumpengehäuses endet bevorzugt mindestens ein Teil der Pumpengehäusewand. Der Teil des Pumpengehäuses, der über den Innenbereich in die Umgebung hinaus ragt, wird als Pumpengehäusezunge bezeichnet. In einer bevorzugten Ausgestaltung der erfindungsgemäßen Pumpvorrichtung weist das Pumpengehäuse an dem ersten Ende, dem Einlass, eine erste Öffnung zu dem Innenbereich und an dem weiteren Ende, dem Auslass, eine weitere Öffnung zu dem Innenbereich auf. Über Einlass und Auslass ist das Pumpengehäuse mit seiner Umgebung fluidleitend verbunden. Die Öffnungen an den Enden des Pumpengehäuses ermöglichen ein Durchfließen eines Fluids durch den Innenbereiches des Pumpengehäuses. Das Fluid ist beispielsweise ein Gas, eine Flüssigkeit, wie Blut, oder einer Mischung hieraus. Bevorzugt dient die erste Öffnung als Zuleitung des zu fördernden Fluids in den Innenbereich des Pumpengehäuses und die weitere Öffnung als Ableitung des zu fördernden Fluids. Das Pumpengehäuse kann weitere Öffnungen aufweisen, beispielsweise in der Wand des Pumpengehäuses. Diese weiteren Öffnungen können zum zusätzlichen Zuleiten von Fluid oder auf der anderen Seite zum verzweigten Ableiten von Fluid dienen. Wird die erfindungsgemäße Pumpvorrichtung in einen Körper implantiert, um beispielsweise den Blutkreislauf zu unterstützen und damit das Herz zu entlasten, so wird die erfindungsgemäße Pumpvorrichtung über Leitungen an Blutgefäße des Körpers angeschlossen.

Das Pumpengehäuse beinhaltet mindestens einen Querschnitt, der bevorzugt ausgewählt ist aus der Gruppe bestehend aus kreisförmig, rechteckig oder vieleckig oder ellipsoid. Bevorzugt weist das Pumpengehäuse eine längliche Form mindestens in einem ersten Abschnitt auf. Weiterhin kann das Pumpengehäuse mindestens einen weiteren Abschnitt beinhalten, dessen Form von dem ersten Abschnitt des Pumpengehäuses abweicht.

Bevorzugt ist die Gesamtlänge des Pumpengehäuses 1,5- bis 10-mal, bevorzugt 2- bis 9-mal, oder bevorzugt 2,5- bis 8,5-mal länger als der Durchmesser des Pumpengehäuses. Die Länge des Pumpengehäuses wird bevorzugt entlang der Außenwand des Pumpengehäuses in Pumprichtung bestimmt. Das Pumpengehäuse weist bevorzugt eine Länge in einem Bereich von 1 mm bis 10 cm, oder bevorzugt in einem Bereich von 2 mm bis 8 cm, oder bevorzugt in einem Bereich von 5 mm bis 5 cm auf. Das Pumpengehäuse weist bevorzugt einen Innendurchmesser in einem Bereich von 0,1 bis 50 mm, oder bevorzugt in einem Bereich von 0,5 bis 30 mm, oder bevorzugt in einem Bereich von 1 bis 20 mm auf.

Die Wand, insbesondere die mindestens eine Wandfläche des Pumpengehäuses, ist bevorzugt glatt. Glatt bedeutet, dass die Wand des Pumpengehäuses eine Rauheit in einem Bereich von 0,025 bis 4 Ra, oder bevorzugt in einem Bereich von 0,05 bis 3 Ra, oder bevorzugt in einem Bereich von 0,07 bis 1 Ra aufweist.

Das Pumpengehäuse beinhaltet mindestens einen ersten Teilbereich und mindestens einen weiteren Teilbereich. Der erste und der weitere Teilbereich unterscheiden sich durch ihre Zusammensetzung. Der mindestens eine erste Teilbereich weist bevorzugt mindestens eine, besonders bevorzugt alle der folgenden Eigenschaften auf:
- möglichst hohe thermische Beständigkeit;
- möglichst hohe Druckbeständigkeit;
- möglichst hohe Härte;
- möglichst hohe Beständigkeit gegen Säuren und Basen;
- möglichst geringe Rauigkeit;
- möglichst spannungsfreie Verbindbarkeit mit einem Metall oder einem Metall-Keramik-Gemisch (Cermet);
- möglichst gute Versinterbarkeit mit einem Metall oder einem Metall-Keramik-Gemisch;
- möglichst geringe elektrische Leitfähigkeit;
- möglichst geringe magnetische Permeabilität.

Der mindestens eine weitere Teilbereich weist bevorzugt mindestens eine, besonders bevorzugt alle der folgenden Eigenschaften auf:
- möglichst hohe thermische Beständigkeit;
- möglichst hohe Druckbeständigkeit;
- möglichst hohe Beständigkeit gegen Säuren und Basen;
- möglichst geringe Rauigkeit;
- möglichst gute Versinterbarkeit mit einem keramischen Material;
- möglichst gute Verbindbarkeit mit einem Metall;
- möglichst gute Verschweißbarkeit mit einem Metall.

Werden die beiden ersten und weiteren Teilbereiche bei der Herstellung des Pumpengehäuses zusammengebracht, so kann ein Pumpengehäuse erhalten werden, das die für den mindestens einen ersten Teilbereich und den mindestens einen weiteren Teilbereich eine oder mehrere der aufgelisteten Eigenschaften vereinigt. Mindestens ein Teil des mindestens einen ersten Teilbereiches ist mit mindestens einem Teil des weiteren Teilbereiches verbunden. Die Verbindung kann eine unmittelbare Verbindung der beiden Teilbereiche sein oder eine mittelbare. Sind der mindestens eine erste Teilbereich und der mindestens eine weitere Teilbereich unmittelbar miteinander verbunden, so sind der erste Teilbereich und der weitere Teilbereich stoffschlüssig miteinander verbunden.

Eine stoffschlüssige Verbindung liegt vor, wenn die stofflichen Eigenschaften des ersten Teilbereiches fließend in die stofflichen Eigenschaften des weiteren Teilbereiches übergehen. Es liegt keine scharfe Grenze zwischen den beiden Teilbereichen vor. Vielmehr besteht ein Übergangsbereich in dem sich die Eigenschaften der beiden Teilbereiche mischen. Dieser Übergangsbereich wird auch als dritter Teilbereich bezeichnet. In diesem dritten Teilbereich liegen sowohl die Materialien des ersten Teilbereiches zum Teil als auch die Materialien des weiteren Teilbereiches nebeneinander vor und bilden bevorzugt eine Gemengelage der Materialien. Bevorzugt gehen die Materialien der beiden Teilbereiche Verbindungen auf atomarer oder molekularer Ebene ein. Es wirken Kräfte auf atomarer oder molekularer Ebene der Materialien der ersten und weiteren Teilbereiche. Eine solche stoffschlüssige Verbindung kann in der Regel nur durch Zerstörung des Pumpengehäuses gelöst werden.

Der mindestens eine erste Teilbereich beinhaltet zu mindestens 61 Gew.-%, bevorzugt zu mindestens 70 Gew.-%, oder bevorzugt zu mindestens 90 Gew.-%, bezogen auf die Gesamtmasse des ersten Teilbereiches eine Keramik. Bevorzugt beinhaltet der mindestens eine erste Teilbereich die Keramik in einem Bereich von 61 bis 100 Gew.-%, oder bevorzugt in einem Bereich von 70 bis 100 Gew.-%, oder bevorzugt in einem Bereich von 80 bis 100 Gew.-%, bezogen auf die Gesamtmasse des ersten Teilbereiches. Weiterhin bevorzugt beinhaltet der mindestens eine erste Teilbereich die Keramik zu 100 Gew.-%, bezogen auf die Gesamtmasse des ersten Teilbereiches. Der mindestens eine erste Teilbereich kann weitere Materialien beinhalten. Die weiteren Materialien können ausgewählt sein aus der Gruppe bestehend aus Wasser, einem Additiv, einem Cermet oder einer Mischung von mindestens zwei hiervon.

Die Keramik kann jede Keramik sein, die der Fachmann für die erfindungsgemäße Pumpvorrichtung auswählen würde. Die Keramik ist bevorzugt ausgewählt aus der Gruppe bestehend aus einer Oxidkeramik, einer Silikatkeramik, einer Nichtoxid-Keramik oder einer Mischung aus mindestens zwei davon.

Die Oxidkeramik ist bevorzugt ausgewählt aus der Gruppe bestehend aus einem Metalloxid, einem Halbmetalloxid oder einer Mischung davon. Das Metall des Metalloxids kann ausgewählt sein aus der Gruppe bestehend aus Aluminium, Beryllium, Barium, Calcium, Magnesium, Natrium, Kalium, Eisen, Zirkonium, Titan oder einer Mischung von mindestens zwei davon. Das Metalloxid ist bevorzugt ausgewählt aus der Gruppe bestehend aus Aluminiumoxid (Al₂O₃), Magnesiumoxid (MgO), Zirkoniumoxid (ZrO₂), Yttriumoxid (Y₂O₃), Aluminiumtitanat (Al2TiO₅), einer Piezokeramik wie Blei-Zirkonat (PbZrO₃), Blei-Titanat (PbTiO₃) sowie Blei-Zirkonat-Titanat (PZT) oder einer Mischung von mindestens zwei hiervon. Das Halbmetall des Halbmetalloxids ist bevorzugt ausgewählt aus der Gruppe bestehend aus Bor, Silicium, Arsen, Tellur oder einer Mischung von mindestens zwei davon.

Die Silikatkeramik ist bevorzugt ausgewählt aus der Gruppe bestehend aus einem Steatit (Mg₃[Si₄O₁₀(OH)₂]), Cordierit (Mg, Fe²⁺)₂(Al₂Si)[Al₂Si₄O₁₈]), Mullit (Al₂Al₂₊₂ₓSi₂₋₂ₓO₁₀₋ₓ mit x = Sauerstoffleerstellen pro Elementarzelle), Feldspat (Ba,Ca,Na,K,NH₄)(Al,B,Si)₄O₈) oder einer Mischung aus mindestens zwei davon.

Die Nichtoxid-Keramik kann ausgewählt sein aus der Gruppe bestehend aus einem Carbid, einem Nitrid oder einer Mischung daraus. Das Carbid kann ausgewählt sein aus der Gruppe bestehend aus Siliciumcarbid (SiC), Borcarbid (B₄C), Titancarbid (TiC), Wolframcarbid, Zementit (Fe3C). Das Nitrid kann ausgewählt sein aus der Gruppe bestehend aus Siliciumnitrid (Si₃N₄), Aluminiumnitrid (AIN), Titannitrid (TiN), Siliciumaluminiumoxinitrid (SIALON) oder einer Mischung aus mindestens zwei davon.

Im Rahmen der Erfindung wird als "Cermet" ein Verbundwerkstoff aus einem oder mehreren keramischen Werkstoffen in mindestens einer metallischen Matrix oder ein Verbundwerkstoff aus einem oder mehreren metallischen Werkstoffen in mindestens einer keramischen Matrix verstanden. Zur Herstellung eines Cermets kann beispielsweise ein Gemisch aus mindestens einem keramischen Pulver und mindestens einem metallischen Pulver verwendet werden, welches beispielsweise mit mindestens mit einem Bindemittel und gegebenenfalls mindestens einem Lösungsmittel versetzt werden kann. Eine Auswahl für die keramischen Bestandteile und die metallischen Bestandteile des Cermets können sich aus denen zusammensetzen, die für den ersten Teilbereich angegeben wurden.

Der mindestens eine erste Teilbereich und der mindestens eine weitere Teilbereich können auf unterschiedliche Weise innerhalb des Pumpengehäuses angeordnet sein. Bevorzugt weist mindestens eine Oberfläche des mindestens einen weiteren Teilbereiches zur Außenseite des Pumpengehäuses hin und mindestens eine Oberfläche des mindestens einen ersten Teilbereichs zum Innenbereich. Der mindestens eine erste Teilbereich oder der mindestens eine weitere Teilbereich kann die gesamte Wandstärke in einem Querschnitt des Pumpengehäuses bilden. Alternativ kann ein Teil der Wandstärke in einem Querschnitt den ersten Teilbereich beinhalten und der andere Teil dieser Wandstärke mindestens einen weiteren Teilbereich beinhalten. Bevorzugt sind der mindestens eine erste Teilbereich und der mindestens eine weitere Teilbereich als Abschnitte des Pumpengehäuses ausgestaltet, die sich entlang der Längsausdehnung des Pumpengehäuses nacheinander erstrecken. Weist das Pumpengehäuse einen dritten Teilbereich auf, so erstreckt sich dieser bevorzugt durch die komplette Wanddicke des Pumpengehäuses an dieser Stelle.

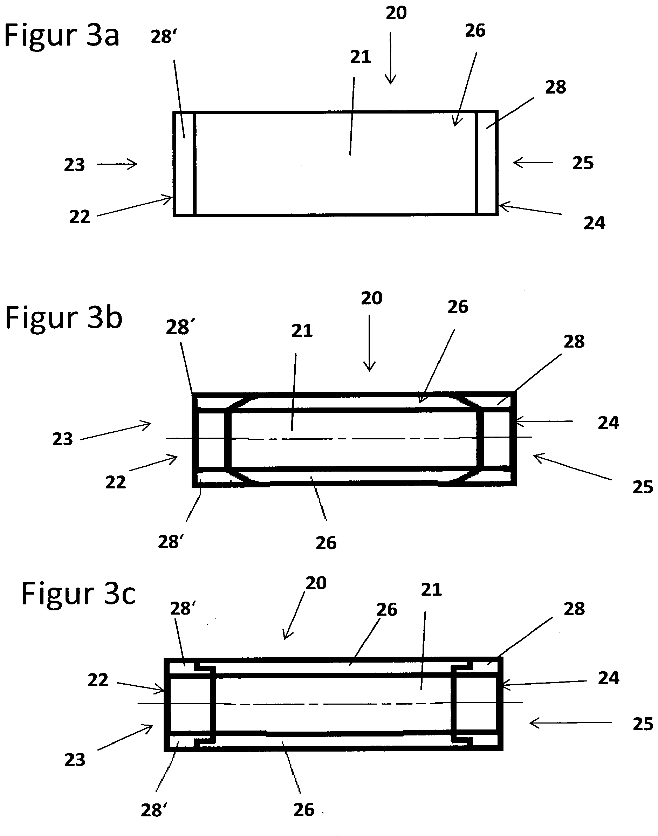

Jeder Übergang von einem Teilbereich zu einem anderen Teilbereich kann in Bezug auf einen Querschnitt des Pumpengehäuses rechtwinklig oder in einem von 90 ° verschiedenen Winkel angeordnet sein. Ferner kann jeder Übergang auch unregelmäßig ausgebildet sein, d.h. im Querschnitt kann keine gedachte grade Linie auf dem Übergang angelegt werden. Weiterhin kann jeder Übergang von einem Teilbereich zu einem anderen Teilbereich alternativ oder zusätzlich zu dem vorstehend beschriebenen in Bezug auf einen Längsschnitt durch eine Wand des Pumpengehäuses rechtwinklig oder in einem von 90 ° verschiedenen Winkel angeordnet sein. Ferner kann jeder Übergang auch unregelmäßig ausgebildet sein, d.h. im Längsschnitt kann keine gedachte grade Linie auf dem Übergang angelegt werden. Ferner sind Kombinationen der vorstehend genannten Konfigurationen eines Übergangs im Querschnitt und im Längsschnitt bevorzugt. Beispiele hierzu sind in Figur 3a bis 3c gezeigt.

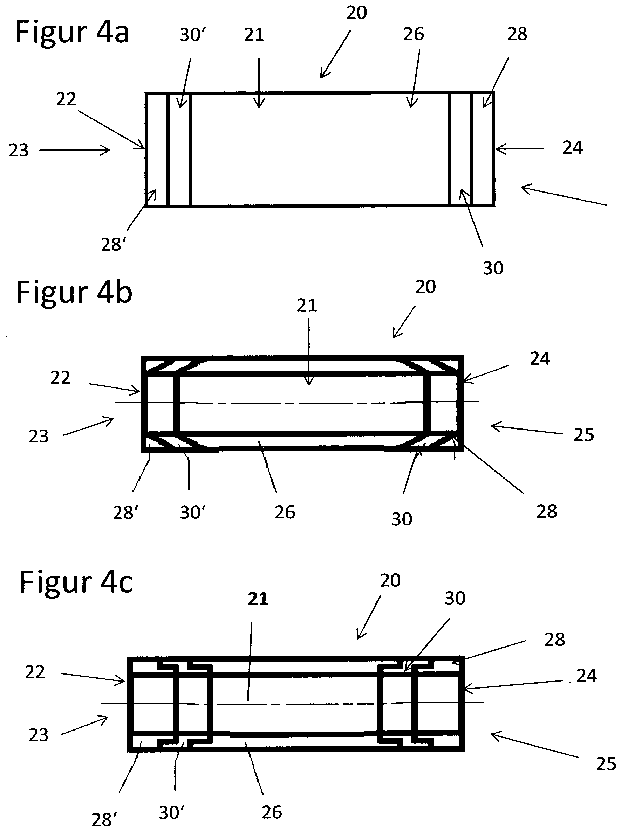

Der mindestens eine erste, eine weitere oder eine dritte Teilbereich kann sich in einer bevorzugten Ausgestaltung des Pumpgehäuses zumindest einem Teil schräg zur Längsausdehnung des Pumpengehäuses erstrecken. Aufgrund dessen kann das Pumpengehäuse Querschnitte des Pumpengehäuses aufweisen, die entlang der Wandstärke sowohl einen ersten als auch einen weiteren Teilbereich, oder sowohl einen ersten als auch einen dritten Teilbereich aufweisen. Dies ist beilspielhaft in Figur 4b dargestellt.

Die erfindungsgemäße Pumpvorrichtung beinhaltet zudem einen Rotor in Form des Impellers. Der Impeller kann jede Form aufweisen, die der Fachmann hierfür auswählen würde.

Der Impeller weist bevorzugt einen Durchmesser in einem Bereich von 1 mm bis 10 cm, bevorzugt in einem Bereich von 3 mm bis 5 cm, oder bevorzugt in einem Bereich von 5 mm bis 3 cm auf. Der Impeller weist bevorzugt eine Dicke in einem Bereich von 0,1 bis 50 mm, bevorzugt in einem Bereich von 0,5 bis 20 mm, oder bevorzugt in einem Bereich von 1 bis 15 mm auf. Der Durchmesser des Impellers ist bevorzugt kleiner als der Durchmesser des Pumpengehäuses in der Ebene des Impellers. Der Durchmesser des Impellers ist bevorzugt in einem Bereich von 1 bis 10 %, oder bevorzugt in einem Bereich von 1,5 bis 8 %, oder bevorzugt in einem Bereich von 2 bis 7 %, bezogen auf den Durchmesser des Pumpengehäuses in der Ebene des Impellers, kleiner als der Durchmesser des Pumpengehäuses.

Der Impeller weist bevorzugt mindestens zwei Rotorblätter auf, bevorzugt mindestens drei Rotorblätter, oder bevorzugt mindestens fünf Rotorblätter. Besonders bevorzugt weist der Impeller eine Anzahl von Rotorblättern in einem Bereich von 2 bis 20, bevorzugt in einem Bereich von 5 bis 15, oder bevorzugt in einem Bereich von 8 bis 13 auf. Der Impeller weist bevorzugt eine zentrale Drehachse auf, um die der Impeller gedreht werden kann. Die Drehachse wird auch als Rotationsachse bezeichnet. Die mindestens zwei Rotorblätter sind bevorzugt symmetrisch um die Drehachse des Impellers angeordnet. Der Impeller ist bevorzugt im Innerbereich des Pumpengehäuses angeordnet, wobei die Rotationsachse des Impellers parallel zur Längsausdehnung der Wand des Rohres vorgesehen ist.

Der Impeller kann aus jedem Material hergestellt sein, das der Fachmann für einen Einsatz in der erfindungsgemäßen Pumpvorrichtung auswählen würde. Bevorzugt weist der Impeller mindestens zwei Bereiche auf: Einen ersten Bereich im Zentrum des Impellers um die Rotationsachse herum. Dieser erste Bereich wird auch Kernbereich genannt. Einen zweiten Bereich, auch Rotorbereich genannt. Dieser zweite Bereich weist mindestens zwei Rotorblätter auf, die zur Förderung des zu fördernden Fluids geeignet sind.

Der Impeller beinhaltet mindestens ein Element, wobei das Element hartmagnetische Eigenschaften aufweist. Eine hartmagnetische Eigenschaft bedeutet, dass ein Material eine dauerhafte Magnetisierung in Folge von Aussetzen dieses Materials in einem Magnetfeld erhält. Die Stärke eines magnetisierenden Feldes wird in Abhängigkeit von der Zusammensetzung des Elementes gewählt. Die dazu notwendigen Überlegungen und Berechnungen sind dem Fachmann geläufig. Bevorzugt wird beim Magnetisieren die Induktion des Impellers gesättigt. Nach Abfall des Magnetfeldes besteht die Magnetisierung des hartmagnetischen Materials weiter. Materialien mit hartmagnetischen Eigenschaften können als Dauermagnete eingesetzt werden. Das mindestens eine Elemente ist bevorzugt so an dem Impeller angeordnet, dass es den Impeller bewegt, wenn es alternierend von zwei voneinander unabhängigen elektrischen bzw. magnetischen Feldern angezogen oder abgestoßen wird. Der Impeller beinhaltet bevorzugt mindestens zwei Elemente mit hartmagnetischen Eigenschaften. Weiterhin kann durch mindestens ein optionales Element der Impeller in seiner radialen aber auch axialen Ausrichtung gesteuert werden. Bevorzugt werden die Elemente mit hartmagnetischen Eigenschaften dazu genutzt, den Impeller ohne weitere Hilfsmittel, wie Lagerungen oder sonstige Fixierungen in dem Pumpengehäuse möglichst kontaktlos im Pumpengehäuse zu lagern. Dies ermöglicht einen besonders reibungsarmen und besonders verschleißarmen Betrieb.

Das mindestens eine Element kann beispielsweise durch mindestens ein Rotorblatt verwirklicht werden, das ein hartmagnetisches Material beinhaltet. Alternativ kann an mindestens einem Rotorblatt ein hartmagnetisches Element angeordnet sein. Bevorzugt ist das hartmagnetische Element im Kern des Impellers vorgesehen. Das mindestens eine hartmagnetische Element beinhaltet bevorzugt mindestens ein magnetisierbares Material, wie Eisen, Kobalt, Nickel, Chromdioxid oder eine Mischung von mindestens zwei hiervon. Das mindestens eine Element kann beispielsweise in Form einer Beschichtung aus hartmagnetischem Material auf mindestens einem Rotorblatt oder im Innern des Impellers angeordnet sein. Bevorzugt beinhalten mindestens 50 %, oder bevorzugt mindestens 70 %, oder bevorzugt 100 % der Rotorblätter ein hartmagnetisches Material. Bevorzugt beinhaltet das Element zu mindestens 10 Gew.-%, oder bevorzugt zu mindestens 20 Gew.-%, oder bevorzugt zu mindestens 30 Gew.-%, bezogen auf die Gesamtmasse des Elementes ein hartmagnetisches Metall. Weiterhin bevorzugt beinhaltet das Element eine Kobalt-Chrom-Legierung oder eine Platin-Kobalt-Legierung, insbesondere eine Platin-Kobalt-Legierung (PtCo23) mit einem Anteil an Kobalt von 23 Gew.-% bezogen auf die Gesamtmasse der Legierung, in einem Bereich von 10 bis 100 Gew.-%, oder bevorzugt in einem Bereich von 20 bis 100 Gew.-%, oder bevorzugt in einem Bereich von 30 bis 100 Gew.-%, bezogen auf die Gesamtmasse des Elementes.

Der Impeller kann in seinem Kern, dem Bereich um die Drehachse herum, ein anderes Material aufweisen als in oder an den Rotorblättern. Alternativ kann der Impeller ein einheitliches Material im Kern und den Rotorblättern beinhalten. Das Material der Rotorblätter kann flexibel oder unflexibel sein. Bevorzugt ist das Material des Kerns des Impellers oder der Rotorblätter des Impellers jeweils ausgewählt aus der Gruppe bestehend aus einem Polymer, einem Metall, einer Keramik oder einer Kombination oder Mischung aus mindestens zwei hiervon.

Das Polymer kann ausgewählt sein aus der Gruppe bestehend aus einem Chitosan, einem Fibrin, einem Collagen, einem Caprolacton, einem Lactid, einem Glycolid, einem Dioxanon, einem Polyurethan, einem Polyimid, einem Polyamid, einem Polyester, einem Polymethylmethacrylat, einem Polyacrylat, einem Teflon, einem Copolymer aus mindestens zwei hieraus oder einer Mischung aus mindestens zwei hiervon.

Das Metall kann ausgewählt sein aus der Gruppe bestehend aus Eisen (Fe), Edelstahl, Platin (Pt), Iridium (Ir), Niob (Nb), Molybdän (Mo), Wolfram (W), Titan (Ti), Kobalt (Co), Chrom (Cr), eine Kobalt-Chrom-Legierung, Tantal (Ta), Vanadium (V) und Zirkonium (Zr) oder einer Mischung aus mindestens zwei hiervon, wobei insbesondere bevorzugt sind Titan, Niob, Molybdän, Kobalt, Chrom, Tantal, Zirkonium, Vanadium und deren Legierungen.

Die Keramik kann ausgewählt sein aus der Gruppe bestehend aus Aluminiumoxid (Al₂O₃), Zirkoniumdioxid (ZrO₂), Hydroxilapatit, Tricalciumphosphat, Glaskeramik, Aluminiumoxid verstärktes Zirkoniumoxid (ZTA), Zirkoniumoxid enthaltendes Aluminiumoxid (ZTA- Zirconia Toughened Aluminum - Al₂O₃/ZrO₂), Yttrium enthaltendes Zirkoniumoxid (Y-TZP), Aluminiumnitrid (AIN), Titannitrid (TiN), Magnesiumoxid (MgO), Piezokeramik, Barium(Zr, Ti)oxid, Barium(Ce, Ti)oxid und Natrium-Kalium-Niobat oder einer Mischung aus mindestens zwei hiervon.

Weiterhin bevorzugt kann der Impeller an seiner Außenseite, insbesondere an der Außenfläche der Rotorblätter, mit einem biokompatiblen Material beschichtet sein. Geeignete biokompatible Materialien werden weiter im Folgenden beschrieben.

Der Impeller ist bevorzugt in dem Innenbereich des Pumpengehäuses angeordnet, das von dem ersten Teilbereich umgeben ist. Der Impeller ist bevorzugt mit seiner Rotationsachse parallel zur Längsausdehnung der Wand angeordnet. Weiterhin kann der Impeller durch ein Magnetfeld in dem Pumpengehäuse ausgerichtet werden. Der Impeller in dem Innenbereich des Pumpengehäuses wird bevorzugt von magnetischen Feldern von elektrischen Spulen an der Au-ßenseite des Pumpengehäuses ausgerichtet. Die Spulen beinhalten bevorzugt ein elektrisch leitendes Material. Bevorzugt ist das elektrisch leitende Material der Spulen ausgewählt aus der Gruppe bestehend aus Eisen (Fe), Kupfer (Cu), Gold (Au), Silber (Ag), Platin (Pt), Palladium (Pd), Titan (Ti), Chrom (Cr), Kobalt (Co), Wolfram (W) oder einer Mischung aus mindestens zwei hiervon. Weiterhin bevorzugt beinhaltet das elektrisch leitende Material Kupfer (Cu). Die erfindungsgemäße Pumpvorrichtung beinhaltet bevorzugt mindestens zwei Spulen, bevorzugt mindestens drei Spulen, oder bevorzugt mindestens vier Spulen. Die Spulen sind bevorzugt an der Außenseite des Pumpengehäuses angeordnet, wobei die Spulen und der Impeller bevorzugt in einer Ebene liegen. Sie sind dann an der Außenseite des Pumpengehäuses um den Impeller herum angeordnet.

In einer bevorzugten Ausgestaltung der erfindungsgemäßen Pumpvorrichtung beinhaltet das Pumpengehäuse ein Rohr. Bevorzugt ist das Rohr gerade. Alternativ kann das Rohr mindestens eine Biegung aufweisen. Das Rohr ist bevorzugt bis auf einen Einlass wie einen Auslass geschlossen. Das bedeutet, dass das Rohr außer den beiden Öffnungen am Einlass und Auslass keine weiteren Öffnungen aufweist. Die Dimensionen, Materialien und Ausgestaltungen entsprechen bevorzugt ansonsten denen des zuvor beschriebenen Pumpengehäuses.

In einer bevorzugten Ausgestaltung der erfindungsgemäßen Pumpvorrichtung ist mindestens ein weiterer Teilbereich an dem Einlass oder dem Auslass vorgesehen.

In einer anderen bevorzugten Ausgestaltung der erfindungsgemäßen Pumpvorrichtung sind je ein weiterer Teilbereich an dem Einlass und dem Auslass vorgesehen.

In einer bevorzugten Ausgestaltung der erfindungsgemäßen Pumpvorrichtung weist das Pumpengehäuse ein Volumen in einem Bereich von 0,1 cm³ bis 10 cm³, bevorzugt in einem Bereich von 0,2 bis 9 cm³, oder bevorzugt in einem Bereich von 0,5 bis 5 cm³ auf. Bevorzugt sind die Dimensionen wie Länge, Durchmesser und Wanddicke des Pumpengehäuses wie bereits oben angegeben. Das Volumen des Pumpengehäuses ist durch den vom Pumpengehäuse umgebenen Innenraum definiert. Die Wand des Pumpengehäuses weist bevorzugt eine Stärke bzw. Dicke in einem Bereich von 0,1 bis 5 mm, oder bevorzugt in einem Bereich von 0,3 bis 4 mm, oder bevorzugt in einem Bereich von 0,4 bis 3 mm auf. Im Folgenden wird in diesem Zusammenhang entweder von Wandstärke oder Wanddicke gesprochen. An der Innenfläche des Pumpengehäuses können in mindestens einem der ersten oder der weiteren Teilbereiche die Wanddicken variieren. Eine Erhöhung der Wanddicke an mindestens einem Punkt des Pumpengehäuses kann dazu dienen, den Impeller mindestens in eine Richtung an seiner Position im Pumpengehäuse zu halten.

In einer bevorzugten Ausgestaltung der erfindungsgemäßen Pumpvorrichtung ist die Keramik des mindestens einen ersten Teilbereiches ausgewählt aus der Gruppe bestehend aus Aluminiumoxid (Al₂O₃), Zirkoniumdioxid (ZrO₂), einem ein Aluminiumoxid enthaltendes Zirkoniumoxid (ATZ - Aluminum toughened Zirconia), einem ein Zirkoniumoxid enthaltendes Aluminiumoxid (ZTA - Zirconia Toughened Aluminum), einem ein Yttrium enthaltendes Zirkoniumoxid (Y-TZP), Aluminiumnitrid (AIN), Titannitrid (TiN), Magnesiumoxid (MgO), einer Piezokeramik, Barium(Zr, Ti)oxid, Barium(Ce, Ti)oxid und Natrium-Kalium-Niobat oder einer Mischung aus mindestens zwei hiervon.

Der mindestens eine weitere Teilbereich des Pumpengehäuses beinhaltet einen Metallgehalt in einem Bereich von 40 bis 90 Gew.-%, bevorzugt in einem Bereich von 45 bis 85 Gew.-%, oder in einem Bereich von 50 bis 80 Gew.-%, bezogen auf die Gesamtmasse des weiteren Teilbereiches.

In einer bevorzugten Ausgestaltung der erfindungsgemäßen Pumpvorrichtung ist das Metall des weiteren Teilbereiches ausgewählt aus der Gruppe bestehend Platin (Pt), Eisen (Fe), Edelstahl (AISI 304, AISI 316 L) Iridium (Ir), Niob (Nb), Molybdän (Mo), Wolfram (W), Titan (Ti), Kobalt (Co), Chrom (Cr), eine Kobalt-Chrom-Legierung, Tantal (Ta) und Zirkonium (Zr) oder einer Mischung aus mindestens zwei hiervon. Bevorzugt ist das Metall ausgewählt aus der Gruppe bestehen aus Titan, Niob, Molybdän, Kobalt, Chrom, Tantal und deren Legierungen oder einer Mischung aus mindestens zwei hiervon.

Weiterhin kann der mindestens eine weitere Teilbereich weitere Materialien aufweisen. Das weitere Material kann, wie bereits für den ersten Teilbereich beschrieben, ausgewählt sein aus der Gruppe bestehend aus einem Additiv, einer Keramik, einem Cermet oder einer Mischung aus mindestens zwei hiervon.

Die Keramik des weiteren Teilbereichs kann jede Keramik sein, die der Fachmann für eine Pumpvorrichtung auswählen würde. Die Keramik ist bevorzugt ausgewählt aus der Gruppe bestehend aus einer Oxidkeramik, einer Silikatkeramik, einer Nichtoxid-Keramik oder einer Mischung aus mindestens zwei davon. Die Keramik des mindestens einen weiteren Teilbereiches kann aus der gleichen Gruppe ausgewählt sein wie die für den ersten Teilbereich aufgeführten Keramiken. Bevorzugt weist der mindestens eine weitere Teilbereich die gleiche Keramik auf wie der mindestens eine weitere Teilbereich. Der weitere Teilbereich beinhaltet die Keramik bevorzugt in einem Bereich von 1 bis 60 Gew.-%, oder bevorzugt in einem Bereich von 5 bis 55 Gew.-%, oder bevorzugt in einem Bereich von 10 bis 45 Gew.-%, bezogen auf die Gesamtmasse des weiteren Teilbereiches.

Weiterhin kann der mindestens eine weitere Teilbereich ein Additiv beinhalten, wie es für den ersten Teilbereich beschrieben wurde. Das Additiv beinhaltet bevorzugt ein Material ausgewählt aus der Gruppe bestehend aus Wasser, einem Dispergiermittel, einem Bindemittel oder einer Mischung von mindestens zwei hiervon. Alle weiteren Eigenschaften und Mengenangaben zu den Additiven können den Angaben zu dem ersten Teilbereich entnommen werden. Die Summe aller Bestandteile des weiteren Teilbereiches ergibt stets 100 Gew.-%.

Eine Auswahl für die keramischen Bestandteile und die metallischen Bestandteile des Cermets können sich aus denen zusammensetzen, die für den mindestens einen weiteren Teilbereich angegeben wurden.

Das Pumpengehäuse beinhaltet bevorzugt mindestens einen ersten Teilbereich und mindestens einen weiteren Teilbereich. Das Pumpengehäuse kann mehrere erste Teilbereiche und mehrere weitere Teilbereiche aufweisen. Bevorzugt weist das Pumpengehäuse eine Anzahl an ersten Teilbereichen in einem Bereich von 1 bis 10, bevorzugt von 1 bis 8, oder bevorzugt von 1 bis 5 auf. Bevorzugt weist das Pumpengehäuse eine Anzahl an weiteren Teilbereichen in einem Bereich von 1 bis 10, bevorzugt von 2 bis 8, oder bevorzugt von 2 bis 5 auf. Bevorzugt beinhaltet das Pumpengehäuse einen ersten Teilbereich und zwei weitere Teilbereiche. Der mindestens eine erste und der mindestens eine weiter Teilbereich können gleich groß sein oder alternativ unterschiedliche Größen aufweisen. Der mindestens eine erste Teilbereich und der mindestens eine weitere Teilbereich erstrecken sich bevorzugt über die gesamte Dicke der Pumpengehäusewand. Die Dicke der ersten und weiteren Teilbereich sind bevorzugt zueinander identisch und entsprechen der Wanddicke wie für die Pumpengehäusewand oben angegeben. Der mindestens eine erste Teilbereich weist bevorzugt eine Breite, bezogen auf die Längsausdehnung des Pumpengehäuses, in einem Bereich von 1 bis 100 mm, bevorzugt in einem Bereich von 2 bis 70 mm, oder bevorzugt in einem Bereich von 3 bis 50 mm auf. Der mindestens eine weitere Teilbereich weist bevorzugt eine Breite bezogen auf die Längsausdehnung des Pumpengehäuses, in einem Bereich von 0,5 bis 80 mm, bevorzugt in einem Bereich von 1 bis 60 mm, oder bevorzugt in einem Bereich von 2 bis 20 mm auf. Weist das Pumpengehäuse mehr als einen ersten und/oder mehr als einen weiteren Teilbereich auf, so sind die mehreren ersten und/oder mehreren weiteren Teilbereiche bevorzugt gleich breit. Alternativ können sich unterschiedliche breite erste und unterschiedlich breite weitere Teilbereiche abwechseln.

In einer bevorzugten Ausführungsform der erfindungsgemäßen Pumpvorrichtung beinhaltet das Pumpengehäuse an seinen beiden Enden jeweils einen gleich großen weiteren Teilbereich. Diese stehen über einen ersten Teilbereich in Verbindung. Bevorzugt weisen die beiden weiteren Teilbereiche eine Breite in einem Bereich von 1 bis 10 mm, oder bevorzugt in einem Bereich von 2 bis 8 mm, oder bevorzugt in einem Bereich von 2,5 bis 6 mm auf. Weiterhin bevorzugt weist der eine erste Teilbereich eine Breite von 5 bis 40 mm, bevorzugt in einem Bereich von 10 bis 30 mm, oder bevorzug in einem Bereich von 15 bis 25 mm auf.

In einer weiteren bevorzugten Ausführungsform der erfindungsgemäßen Pumpvorrichtung beinhaltet das Pumpengehäuse an seinen beiden Enden jeweils einen weiteren Teilbereich, wobei die beiden weiteren Teilbereiche durch einen ersten Teilbereich getrennt sind. Bevorzugt weisen die beiden weiteren Teilbereiche eine Breite in einem Bereich von 3 bis 30 mm, oder bevorzugt in einem Bereich von 5 bis 20 mm, oder bevorzugt in einem Bereich von 7 bis 15 mm auf. Weiterhin bevorzugt weist der eine erste Teilbereich eine Breite von 0,5 bis 10 mm, oder bevorzugt in einem Bereich von 1 bis 8 mm, oder bevorzugt in einem Bereich von 2 bis 6 mm auf. Die beiden weiteren Teilbereiche, je einer am Einlass und am Auslass, können gleiche oder unterschiedliche Breiten, Durchmesser und Wandstärken aufweisen. Bevorzugt weist das Pumpengehäuse am Einlass und/oder Auslass mindestens einen unterschiedlichen Innendurchmesser gegenüber dem sonstigen Innendurchmessers des Pumpengehäuses auf. Der unterschiedliche Innendurchmesser kann entweder durch unterschiedlich dicke Wandstärken oder durch unterschiedliche Anordnung bzw. Geometrie der weiteren Teilbereiche an dem ersten Teilbereich erreicht werden.

Weiterhin kann der mindestens eine erste Teilbereich ein Metall beinhalten. Das Metall kann jedes Metall sein, das der Fachmann für die Fertigung des Pumpengehäuses auswählen würde.

In einer bevorzugten Ausgestaltung der erfindungsgemäßen Pumpvorrichtung beinhaltet der mindestens eine erste Teilbereich weniger als 10 Gew.-%, bevorzugt von weniger als 5 Gew.-%, oder bevorzugt von weniger als 3 Gew.-%, bezogen auf die Gesamtmasse des ersten Teilbereiches, an Metall. Die Summe aller Bestandteile des ersten Teilbereiches ergibt stets 100 Gew.-%.

Das Metall des ersten Teilbereiches ist bevorzugt ausgewählt aus der Gruppe bestehend aus Platin (Pt), Eisen (Fe), Edelstahl (AISI 304, AISI 316 L), Iridium (Ir), Niob (Nb), Molybdän (Mo), Wolfram (W), Titan (Ti), Kobalt (Co), Chrom (Cr), eine Kobalt-Chrom-Legierung, Tantal (Ta), und Zirkonium (Zr) oder einer Mischung aus mindestens zwei hiervon. Bevorzugt ist das Metall ausgewählt aus der Gruppe bestehen aus Titan, Niob, Molybdän, Kobalt, Chrom, Tantal und deren Legierungen oder einer Mischung aus mindestens zwei hiervon. Bevorzugt weist der erste Teilbereich das gleiche Metall auf wie der weitere Teilbereich.

In einer bevorzugten Ausgestaltung der erfindungsgemäßen Pumpvorrichtung sind der mindestens eine erste Teilbereich und der mindestens eine weitere Teilbereich durch mindestens einen dritten Teilbereich miteinander verbunden. Bevorzugt ist jeder erste mit jedem weiteren Teilbereich über einen dritten Teilbereich verbunden.

In einer bevorzugten Ausgestaltung der erfindungsgemäßen Pumpvorrichtung weist der mindestens eine dritte Teilbereich einen Metallgehalt zwischen dem Metallgehalt des ersten Teilbereiches und dem Metallgehalt des weiteren Teilbereiches auf. Der dritte Teilbereich kann sich aufgrund des Herstellprozesses des Pumpengehäuses zwischen dem mindestens einen ersten und dem mindestens einen weiteren Teilbereich befinden. Alternativ kann beim Herstellungsprozess ein dritter Teilbereich mindestens zwischen einem ersten und einem weiteren Teilbereich eingebracht worden sein. Der dritte Teilbereich beinhaltet bevorzugt eine Keramik und ein Metall. Die Keramik ist bevorzugt ausgewählt aus den für den ersten Teilbereich aufgelisteten Keramiken. Das Metall ist bevorzugt ausgewählt aus den für den weiteren Teilbereich aufgelisteten Metallen. Der dritte Teilbereich beinhaltet die Keramik bevorzugt in einem Bereich von 10 bis 90 Gew.-%, oder bevorzugt in einem Bereich von 20 bis 80 Gew.-% oder bevorzugt in einem Bereich von 30 bis 70 Gew.-%, bezogen auf die Gesamtmasse des dritten Teilbereiches. Der dritte Teilbereich beinhaltet das Metall bevorzugt in einem Bereich von 10 bis 89 Gew.-%, oder bevorzugt in einem Bereich von 20 bis 80 Gew.-% oder bevorzugt in einem Bereich von 30 bis 70 Gew.-%, bezogen auf die Gesamtmasse des dritten Teilbereiches. Die Summe aller Bestandteile des dritten Teilbereiches ergibt stets 100 Gew.-%. Der dritte Teilbereich weist bevorzugt einen Metallgehalt auf, der sich aus dem Mittelwert des Metallgehaltes des ersten Teilbereiches und des weiteren Teilbereiches ergibt. Der dritte Teilbereich kann dazu dienen Spannungen zwischen den unterschiedlichen Materialien des ersten und des weiteren Teilbereiches abzubauen oder zu minimieren. Bevorzugt ist die Verbindung zwischen dem ersten und dem dritten Teilbereich stoffschlüssig. Weiterhin bevorzugt ist die Verbindung zwischen dem zweiten und dem dritten Teilbereich ebenfalls stoffschlüssig. Bevorzugt weisen der erste, der weitere und der dritte Teilbereich die gleiche Keramik oder die gleichen Keramiken und das gleiche Metall oder die gleichen Metalle auf.

In einer bevorzugten Ausgestaltung der erfindungsgemäßen Pumpvorrichtung ist die Pumpvorrichtung zumindest zu einem Teil von einem Bauteilgehäuse umgeben, wobei mindestens ein Teil des mindestens einen weiteren Teilbereiches der Pumpvorrichtung mit dem Bauteilgehäuse verbunden ist. Die Verbindung des Bauteilgehäuses mit mindestens einem Teil des weiteren Teilbereiches des Pumpengehäuses führt bevorzugt zu einem abgeschlossenen Raum zwischen dem Bauteilgehäuse und dem Pumpengehäuse. Bevorzugt ist das Innere des Bauteilgehäuses der Pumpvorrichtung hermetisch gegen die Umwelt abgeschlossen. Die hier erfindungsgemäß vorgeschlagene medizinisch implantierbare Pumpvorrichtung kann insbesondere in einen Körper eines menschlichen oder tierischen Benutzers, insbesondere eines Patienten, eingesetzt werden. Eine implantierte Pumpvorrichtung ist in der Regel einer Flüssigkeit eines Körpergewebes des Körpers ausgesetzt. Somit ist es in der Regel von Bedeutung, dass weder Körperflüssigkeit in die medizinisch implantierbare Vorrichtung eindringt, noch das Flüssigkeiten aus der medizinisch implantierbaren Vorrichtung austreten. Um dieses sicherzustellen, sollte das Bauteilgehäuse der medizinisch implantierbaren Vorrichtung, und somit auch das Bauteilgehäuse sowie das Pumpengehäuse der erfindungsgemäßen Pumpvorrichtung, eine möglichst vollständige Undurchlässigkeit aufweisen, insbesondere gegenüber Körperflüssigkeiten.

Die erfindungsgemäße Pumpvorrichtung, insbesondere Verbindungen von Bauteilgehäuse mit Pumpengehäuse sind bevorzugt hermetisch dicht. So ist der Innenraum der Pumpvorrichtung hermetisch dicht gegenüber dem Außenraum abgedichtet. Im Rahmen der Erfindung bedeutet der Begriff "hermetisch dicht", dass bei einem bestimmungsgemäßen Gebrauch innerhalb eines üblichen Zeitraums von 5 Jahren keine Feuchtigkeit und/oder Gase die hermetisch dichte Verbindung durchdringen können. Eine physikalische Größe zum Bestimmen der Dichtheit einer Verbindung oder eines Bauteils ist die Leckrate. Dichtheiten können durch Lecktests bestimmt werden. Entsprechende Lecktests werden mit Heliumlecktestern und/oder Massenspektrometern durchgeführt werden und sind im Standard Mil-STD-883G Method 1014 spezifiziert. Die maximal zulässige Helium-Leckrate wird dabei abhängig vom internen Volumen der zu prüfenden Vorrichtung festgelegt. Nach den in MIL-STD-883G, Method 1014, in Absatz 3.1 spezifizierten Methoden, und unter Berücksichtigung der in der Anwendung der vorliegenden Erfindung vorkommenden Volumina und Kavitäten der zu prüfenden Vorrichtungen, beträgt die maximal zulässige Helium-Leckrate für die erfindungsgemäßen Pumpengehäuse 10⁻⁷ atm*cm³/sec oder weniger. Das bedeutet, dass die zu prüfende Vorrichtung (beispielsweise das Bauteilgehäuse und/oder die erfindungsgemäße Pumpvorrichtung oder das Bauteilgehäuse mit dem verbundenen Pumpengehäuse) eine Helium-Leckrate von weniger als 1 x 10⁻⁷ atm*cm³/sec oder weniger aufweist. In einer besonders vorteilhaften Ausführung beträgt die Helium-Leckrate weniger als 1 x 10⁻⁸ atm*cm³/sec, insbesondere weniger als 1 x 10⁻⁹ atm*cm³/sec. Zum Zweck der Standardisierung können die genannten Helium-Leckraten auch in die äquivalente Standard-Luft-Leckrate konvertiert werden. Die Definition für die äquivalente Standard-Luft-Leckrate (Equivalent Standard Air Leak Rate) und die Umrechnung sind im Standard ISO 3530 angegeben.

Die erfindungsgemäße Pumpvorrichtung weist bevorzugt neben dem Impeller, dem Pumpengehäuse mit einem ersten und einem weiteren Teilbereich bevorzugt ein Bauteilgehäuse auf, in dem sich weitere Bauteile der Pumpvorrichtung befinden können. Die weiteren Bauteile der Pumpvorrichtung sind bevorzugt ausgewählt aus der Gruppe bestehend aus einer Batterie, einer Spule, einer Steuereinheit, einer Gefäßverbindungseinheit oder einer Kombination aus mindestens zwei hieraus.

In einer bevorzugten Ausgestaltung der erfindungsgemäße Pumpvorrichtung beinhaltet das Bauteilgehäuse Titan zu mindestens 30 Gew.-%, bevorzugt mindestens 50 Gew.-%, oder bevorzugt mindestens 80 Gew.-%, jeweils bezogen auf die Gesamtmasse des Bauteilgehäuses. Weiter bevorzugt beinhaltet das Bauteilgehäuse Titan zu mindestens 99 Gew.-%, bezogen auf die Gesamtmasse des Bauteilgehäuses. Weiterhin kann das Bauteilgehäuse bevorzugt mindestens ein anderes Metall beinhalten. Das andere Metall kann aus der gleichen Gruppe ausgewählt sein, wie das Metall des weiteren Teilbereiches. Das andere Metall ist bevorzugt ausgewählt aus der Gruppe bestehend aus Fe, Al, V, Sn, Co, Cr, CoCr, Nb, Edelstahl, Mb, TiNb oder einer Mischung von mindestens zwei hiervon. Das Bauteilgehäuse kann das weitere Metall bevorzugt in einem Bereich von 1 bis 70 Gew.-%, oder bevorzugt in einem Bereich von 5 bis 50 Gew.-%, oder bevorzugt in einem Bereich von 10 bis 20 Gew.-% beinhalten. Die Summe aller Bestandteile des Bauteilgehäuses ergibt stets 100 Gew.-%. Geeignete Titanqualitäten sind in ASTM B265-05:2011 angegeben, zum Beispiel Grade 1 bis 6.

In einer bevorzugten Ausgestaltung der erfindungsgemäßen Pumpvorrichtung weist die Wand des Pumpengehäuses eine magnetische Permeabilität von weniger als 2 µ, bevorzugt weniger als 1,9 µ, oder bevorzugt weniger als 1,8 µ auf. Die magnetische Permeabilität wird gemäß der Norm ASTM 773-01:2009 bestimmt.

In einer bevorzugten Ausgestaltung der erfindungsgemäßen Pumpvorrichtung weist die Oberfläche des mindestens einen ersten Teilbereiches, die dem Innenbereich des Pumpengehäuses zugewandt ist, eine Härte nach Vickers von mindestens 330 HV, bevorzugt mindestens 350 HV, oder bevorzugt mindestens 370 HV auf. Bevorzugt weist der gesamte mindestens eine erste Teilbereich eine Härte in den angegeben Bereichen auf. Mindestens die Oberfläche des mindestens einen weiteren Teilbereiches weist ebenfalls eine Härte nach Vickers von mindestens 330 HV, bevorzugt mindestens 350 HV, oder bevorzugt mindestens 370 HV auf. Oftmals ist die Härte nicht höher als 2000 HV, oder bevorzugt nicht höher als 1500 HV. Bevorzugt liegt die Härte mindestens der Oberfläche des mindestens einen ersten Teilbereiches in einem Bereich von 330 bis 2000 HV, oder bevorzugt in einem Bereich von 350 bis 1800 HV. Weiterhin bevorzugt weist mindestens die Oberfläche des mindestens einen ersten Teilbereiches eine Härte auf, die mindestens so groß ist wie die Härte der Rotoroberflächen des Impellers. Bevorzugt weist mindestens die Oberfläche des mindestens einen ersten Teilbereiches eine Härte auf, die um mindestens 20 HV, oder bevorzugt um mindestens 30 HV, oder bevorzugt um mindestens 40 HV höher liegt als die Härte nach Vickers der Rotoroberflächen des Impellers. Als Oberfläche des mindestens einen Teilbereiches, des mindestens einen weiteren Teilbereiches sowie des Impellers wird die oberflächennahe Materieschicht in einem Bereich von 0,01 bis 2,5 mm, bevorzugt in einem Bereich von 0,05 bis 1,0 mm, oder bevorzugt in einem Bereich von 0,1 bis 0,5 mm, jeweils senkrecht zur Oberfläche verstanden.

In einer bevorzugten Ausgestaltung der erfindungsgemäßen Pumpvorrichtung sind zumindest die Außenflächen des Bauteilgehäuses und die dem Innenbereich des Pumpengehäuses zugewandte Oberfläche biokompatibel. Dies ist insbesondere bevorzugt, wenn die Pumpvorrichtung für die Implantation in einen lebenden Körper, wie beispielsweise den eines Menschen oder Tieres. Die Biokompatibilität wird ermittelt und beurteilt gemäß der Norm ISO 10993-4:2002.

In der Regel kommen die dem Innenbereich des Pumpengehäuses zugewandten Oberflächen und die Außenflächen des Bauteilgehäuses nach Implantieren der erfindungsgemäßen Pumpvorrichtung in einen lebenden Körper mit dessen Körperflüssigkeit in Kontakt. Die Biokompatibilität der mit Körperflüssigkeit in Berührung kommenden Oberflächen trägt dazu bei, dass der Körper beim Kontakt mit diesen Oberflächen keinen Schaden nimmt.

Ein Verfahren zur Herstellung eines Pumpengehäuses für eine Pumpvorrichtung kann folgende Schritte beinhalten:
a. Bereitstellen eines ersten Materials;
b. Bereitstellen eines weiteren Materials
c. Bilden eines Pumpengehäusevorläufers, wobei ein erster Teilbereich des Pumpengehäuses aus dem ersten Material und wobei ein weiterer Teilbereich des Pumpengehäuses aus dem weiteren Material gebildet wird;
d. Behandeln des Pumpengehäusevorläufers bei einer Temperatur von mindestens 300 °C.

Das Bereitstellen des ersten Materials in Schritt a. und des weiteren Materials in Schritt b. kann auf jede beliebige Art und Weise erfolgen, die der Fachmann für diesen Zweck auswählen würde.

Das Bilden des Pumpengehäusevorläufers kann auf jede beliebige Art und Weise erfolgen, die der Fachmann für den Zweck der Bildung eines ersten Teilbereiches und eines weiteren Teilbereiches auswählen würde. In einer bevorzugten Ausgestaltung des Verfahrens beinhaltet Schritt c. einen Formgebungsprozess, bevorzugt ausgewählt aus der Gruppe bestehend aus einem lithographischen Prozess, einem Spritzgießen, einem Zerspanen, einem Extrudieren oder einer Kombination von mindestens zwei hiervon.

In einem lithographischen Prozess werden verschiedene Schichten eines oder mehrerer Materialien nacheinander in einer Form gebracht. Der lithographische Prozess entspricht bevorzugt einem schichtweisen Siebdruckverfahren. Beim Siebdruckverfahren wird ein Sieb, bestehend aus einem möglichst formstabilen Material, wie Holz; Metall, bevorzugt Stahl; einer Keramik oder einem Kunststoff mit einer ausgewählten Maschenweite auf das zu überlagernde oder über dem zu überlagernden Objekt, angeordnet. Auf dieses Sieb wird über eine Düse oder aus einem Behälter die zum Aufbringen oder Überlagern verwendete Druckmasse, beispielsweise in Form eine Paste oder eines Pulvers, aufgebracht und mit einer Rakel durch die Maschen des Siebs gedrückt. Dabei kann aufgrund eines Musters in dem Sieb an unterschiedlichen Stellen unterschiedlich viel, zum Aufbringen oder Überlagern verwendete Druckmasse aufgebracht werden. So kann durch die Geometrie und Anordnung der Maschen entweder ein gleichmäßiger Film der zum Überlagern verwendeten Druckmasse aufgebracht werden oder Bereiche mit keiner oder wenig zum Aufbringen verwendeten Druckmasse mit Bereichen mit viel zum Aufbringen verwendeten Druckmasse abwechseln. Bevorzugt wird ein gleichmäßiger Film der zum Überlagern verwendeten Druckmasse auf die Oberfläche übertragen. Die Siebmaschen können auch durch entsprechend aufgebrachte Materialien (Kopierschichten, Siebdruckschablonen) teilweise geschlossen sein, so dass die Druckmasse nur in definierten Bereichen mit offenen Maschen auf die zu beschichtende Oberfläche übertragen wird, um so beispielsweise eine definierte Struktur wie ein Muster zu erhalten. Weiterhin können statt Sieben auch dünne Filme mit definierten Öffnungen (Stencil) zum Übertragen der Druckmasse verwendet werden. Durch Wiederholung dieses Vorgangs mit ein und dem selben Material oder auch unterschiedlichen Materialien können 3-D Strukturen erhalten werden.

Der erste Teilbereich kann bereits als gebrannte Keramik vorliegen. Bevorzugt liegt der erste Teilbereich als ein Rohr mit einer Länge von 2 cm mit einem Innendurchmesser von 9 mm. Auf die Keramik wird dann bevorzugt mindestens eine Schicht des weiteren Materials per Siebdruck an mindestens einem Ende des ersten Teilbereiches aufgebracht wird. Der Vorgang des Siebdruckens wird so lange wiederholt, bis eine Schicht von 5 mm an weiterem Teilbereich auf dem Ende des ersten Teilbereiches aufgebracht ist. Alternativ können alle Teilbereiche nacheinander per Siebdruck hergestellt werden.

Das Spritzgießen, oder auch Spritzgussverfahren genannt, ist ein Formungsprozess für mindestens ein Material, um einen geformten Festkörper zu erhalten. Dem Fachmann sind unterschiedliche Spritzgießverfahren sowie bei Spritzgießen verwendeten Werkzeuge und Bedingungen aus dem Stand der Technik bekannt. Das Spritzgießen kann ausgewählt sein aus der Gruppe bestehend aus einem Mehrkomponenten-Spritzgießen, einem Pulverspritzgießen, einem Spritzprägen, einem Extrusionsspritzgießen, einem Unterdruckspritzgießen oder einer Kombination aus mindestens zwei hiervon.

Das Zerspanen kann mit jedem anderen Formgebungsprozess kombiniert werden. Beim Zerspanen wird ein massiver Körper durch Einsatz von Zerspanungshilfsmitteln, wie einem Bohrer oder einem Stempel strukturiert. Bei dem Strukturieren wird ein Teil des Materials abgetragen. Hierdurch können massive Körper beispielsweise zu Hohlkörpern geformt werden. Beispielsweise kann durch Zerspanen in den Pumpengehäusevorläufer ein Hohlraum geformt werden, wenn der Pumpengehäusevorläufer massiv ausgestaltet ist. Das Zerspanen kann jedoch auch ein Bearbeitungsschritt nach der Herstellung eines Pumpengehäuses oder Gehäuses sein. Zusätzlich zum Zerspanen kann im Anschluss an die Herstellung des Pumpengehäuses auch ein Polieren stattfinden.

Bei dem Bilden des Pumpengehäusevorläufers in Schritt c. wird ein erstes Material zum Bilden eines ersten Teilbereiches mit einem weiteren Material zum Bilden des weiteren Teilbereiches in Kontakt gebracht. Das Inkontaktbringen findet bevorzugt in Form eines Spritzgießens statt, bei dem nacheinander zunächst das weitere Material in eine Form aus Metall gespritzt wird und anschließend das erste Material. Die Mengenverhältnisse im ersten und weiteren Material entsprechen bevorzugt den Mengenverhältnissen im ersten und im weiteren Teilbereich, wie sie oben im Zusammenhang mit dem ersten Gegenstand, der erfindungsgemäßen Pumpvorrichtung, beschrieben wurden. Weiterhin kann das erste und das weitere Material Additive enthalten. Bevorzugt weist der Pumpengehäusevorläufer nach dem Inkontaktbringen bereits die Form des Pumpengehäuses auf. Bevorzugt bilden die beiden Materialien eine kontinuierliche Form. Das Inkontaktbringen kann noch einen oder mehrere weitere Schritte beinhalten. So kann ein drittes Material, das bevorzugt eine Zusammensetzung wie der dritte Teilbereich der zuvor beschriebenen erfindungsgemäßen Pumpvorrichtung, zwischen dem ersten Material und dem weiteren Material in den Pumpengehäusevorläufer eingebracht werden.

Als Additiv kann jede Substanz ausgewählt sein, die der Fachmann als Zusatz für das erste Material auswählen würde. Das Additiv ist bevorzugt ausgewählt aus der Gruppe bestehend aus Wasser, einem Dispergiermittel, einem Bindemittel oder einer Mischung von mindestens zwei hiervon.

Das Dispergiermittel beinhaltet bevorzugt mindestens eine organische Substanz. Die organische Substanz weist bevorzugt mindestens eine funktionale Gruppe auf. Die funktionale Gruppe kann ein hydrophobe oder eine hydrophile funktionale Gruppe sein. Die funktionale Gruppe kann ausgewählt sein aus der Gruppe bestehend aus einer Ammonium-Gruppe, einer Carboxylat-Gruppe, einer Sulfat-Gruppe, einer Sulfonat-Gruppe, einer Alkohol-Gruppe, einer Mehrfachalkohol-Gruppe, einer Ether-Gruppe oder einer Mischung aus mindestens zwei hiervon. Das Dispergiermittel weist funktionale Gruppen bevorzugt in einem Bereich von 1 bis 100, oder bevorzugt in einem Bereich von 2 bis 50, oder bevorzugt in einem Bereich von 2 bis 30 auf. Bevorzugte Dispergiermittel sind unter den Handelsnamen DISPERBYK® 60 von Byk-Chemie GmbH, DOLAPIX CE 64 von Zschimmer & Schwarz GmbH & Co KG erhältlich.

Das Bindemittel ist bevorzugt ausgewählt aus der Gruppe bestehend aus einer Methylcellulose, einem thermoplastischen Polymer, einem duroplastischen Polymer und einem Wachs oder einer Mischung von mindestens zwei hiervon.

Die Methylcellulose ist bevorzugt ausgewählt aus der Gruppe bestehend aus Hydroxypropylmethylcellulose (HPMC), Hydroxyethylmethylcellulose (HEMC), Ethylmethylcellulose (EMC) oder einer Mischung daraus. Die Methylcellulose beinhaltet bevorzugt Hydroxypropylmethylcellulose (HPMC) Weiterhin bevorzugt beinhaltet die Methylcellulose Hydroxypropylmethylcellulose in einem Bereich von 80 bis 100 Gew.-%, oder bevorzugt in einem Bereich von 90 bis 100 Gew.-%, oder bevorzugt in einem Bereich von 95 bis 100 Gew.-%, bezogen auf die Gesamtmasse an Methylcellulose. Bevorzugt weist die Methylcellulose einen Anteil an -OCH₃ Gruppen in einem Bereich von 20 bis 40 Gew.-%, oder bevorzugt in einem Bereich von 23 bis 37 Gew.-%, oder bevorzugt in einem Bereich von 25 bis 35 Gew.-%, bezogen auf die Gesamtmasse an Methylcellulose auf. Weiterhin bevorzugt weist die Methylcellulose einen Anteil an -OC₃H₆OH Gruppen in einem Bereich von 1 bis 12 Gew.-%, oder bevorzugt in einem Bereich von 3 bis 9 Gew.-%, oder bevorzugt in einem Bereich von 4 bis 8 Gew.-%, bezogen auf die Gesamtmasse an Methylcellulose auf.

Das thermoplastische Polymer kann ausgewählt sein aus der Gruppe bestehend aus AcrylnitrilButadien-Styrol (ABS), Polyamide (PA), Polylactat (PLA), Polymethylmethacrylat (PMMA), Polycarbonat (PC), Polyethylenterephthalat (PET), Polyethylen (PE), Polypropylen (PP), Polystyrol (PS), Polyetheretherketon (PEEK) und Polyvinylchlorid (PVC) oder einer Mischung von mindestens zwei davon. Das duroplastische Polymer kann ausgewählt sein aus der Gruppe bestehend aus einem Aminoplasten, einem Epoxidharz, einem Phenolharz, einem Polyester-Harz oder einer Mischung aus mindestens zwei davon. Wachse sind Kohlenwasserstoffverbindungen, die oberhalb 40 °C ohne Zersetzung schmelzen. Hierunter können sich auch Polyester, Paraffine, Polyethylene oder Copolymere aus mindestens zwei daraus befinden.

Der erste Material beinhaltet mindestens eines der vorgenannten Additive bevorzugt in einem Bereich von 0,1 bis 10 Gew.-%, oder bevorzugt in einem Bereich von 0,2 bis 8 Gew.-%, oder bevorzugt in einem Bereich von 0,5 bis 5 Gew.-%, bezogen auf die Gesamtmasse des ersten Materials.

Der weitere Material beinhaltet mindestens eines der vorgenannten Additive bevorzugt in einer Menge in einem Bereich von 0,1 bis 5 Gew.-%, oder bevorzugt in einem Bereich von 0,2 bis 2 Gew.-%, oder bevorzugt in einem Bereich von 0,3 bis 1 Gew.-%, jeweils bezogen auf das Gesamtgewicht des weiteren Materials.

Das Behandeln des Pumpengehäusevorläufers in Schritt d. kann auf jede beliebige Art und Weise erfolgen, die der Fachmann für den Zweck der Erwärmung des Pumpengehäusevorläufers auf mindestens 300 °C auswählen würde. Bevorzugt findet mindestens ein Teil des Behandelns des Pumpengehäusevorläufers bei einer Temperatur in einem Bereich von 300 bis 2500 °C, oder in einem Bereich von 500 bis 2000 °C, oder in einem Bereich von 700 bis 1800 °C statt. Bei der Behandlung des Pumpengehäusevorläufers bei erhöhter Temperatur entweicht bevorzugt mindestens ein Teil des Bindemittels. Es sind verschiedene Temperaturprofile beim Behandeln in Schritt d. des Pumpengehäusevorläufers aus Schritt c. möglich. Das Behandeln des Pumpengehäusevorläufers kann beispielsweise in einer oxidativen Atmosphäre, einer reduktiven Atmosphäre oder unter einer Schutzatmosphäre erfolgen. Eine oxidative Atmosphäre kann beispielsweise Sauerstoff enthalten, wie Luft oder ein Sauerstoff/Luft Gemisch. Eine reduktive Atmosphäre kann beispielsweise Wasserstoff enthalten. Eine Schutzatmosphäre beinhaltet bevorzugt weder Sauerstoff noch Wasserstoff. Beispiele für Schutzatmosphären sind Stickstoff, Helium, Argon, Krypton oder deren Gemische. Die Wahl der Atmosphäre kann abhängig von den zu behandelnden Materialien sein. Dem Fachmann ist die geeignete Wahl der Atmosphäre für die erwähnten Materialien bekannt. Es können auch bevorzugt nacheinander Kombinationen von unterschiedlichen Atmosphären für verschiedene Zeiträume gewählt werden.

Das Behandeln des Pumpengehäusevorläufers kann entweder in einem Schritt erfolgen oder bevorzugt in mehr als einem Schritt. Bevorzugt wird der Pumpengehäusevorläufer in einem ersten Teilschritt des Schrittes d. auf eine Temperatur in einem Bereich von 301 bis 600 °C, oder bevorzugt in einem Bereich von 350 bis 550 °C, oder bevorzugt in einem Bereich von 400 bis 500 °C behandelt. Dieser erste Teilschritt des Behandlungsschrittes d. kann über einen Zeitraum in einem Bereich von 0,1 bis 100 h, bevorzugt in einem Bereich von 1 bis 50 h, oder bevorzugt in einem Bereich von 2 bis 20 h erfolgen. Dieser Teilschritt kann entweder durch Einbringen des Pumpengehäusevorläufers aus Schritt c. in eine vorgeheizte Atmosphäre erfolgen oder durch langsames schrittweises oder stetig erhöhtes Erhitzen des Pumpengehäusevorläufers. Bevorzugt wird das Behandeln in dem ersten Teilschritt des Schritt d. des Pumpengehäusevorläufers in einem Schritt auf eine Temperatur in einem Bereich von 301 bis 600 °C vorgenommen.

In einem zweiten Teilschritt des Behandelns aus Schritt d., der sich bevorzugt an den ersten Teilschritt anschließt, wird der Pumpengehäusevorläufer bevorzugt auf eine Temperatur in einem Bereich von 800 bis 2500 °C, oder bevorzugt in einem Bereich von 1000 bis 2000 °C, oder bevorzugt in einem Bereich von 1100 bis 1800 °C erhitzt. Auch dieser Teilschritt kann entweder durch Einbringen des Pumpengehäusevorläufers aus dem ersten Teilschritt des Schritt d. in eine vorgeheizte Atmosphäre erfolgen oder durch langsames schrittweises oder stetig erhöhtes Erhitzen des Pumpengehäusevorläufers. Bevorzugt wird das Behandeln in dem zweiten Teilschritt des Schritt d. des Pumpengehäusevorläufers in einem Schritt auf eine Temperatur in einem Bereich von 800 bis 2500 °C vorgenommen. Die Behandlung des Pumpengehäusevorläufers in dem zweiten Teilschritt wird über einen Zeitraum in einem Bereich von 0,1 bis 20 h, bevorzugt in einem Bereich von 1 bis 12 h, oder bevorzugt in einem Bereich von 2 bis 5 h vorgenommen.

Die Form des Pumpengehäuses nach dem Herstellungsprozess ist bevorzugt kontinuierlich. Das bedeutet, dass das Pumpengehäuse neben dem Auslass und dem Einlass keine weiteren Öffnungen oder Auslässe, oder sonstige Aussparungen aufweist. Bevorzugt weist das Pumpengehäuse eine geradlinige Außenfläche auf. An der Innenfläche des Pumpengehäuses können in mindestens einem der ersten oder der weiteren Teilbereiche die Wandstärken variieren. Eine Erhöhung der Wandstärke an mindestens einem Punkt des Pumpengehäuses kann dazu dienen, den Impeller mindestens in eine Richtung an seiner Position im Pumpengehäuse zu halten. Die Verdickung der Wandstärke kann dabei entweder bereits während des Herstellprozesses stattfinden oder im Anschluss daran. Ergänzend oder alternativ kann das Pumpengehäuse Einschnürungen aufweisen.

Ein weiteres Verfahren zur Herstellung eines Pumpengehäuses für eine Pumpvorrichtung kann die folgenden Schritte beinhalten:
a) Bereitstellen eines ersten Materials;
b) Bereitstellen eines weiteren Materials
c) Bilden eines Pumpengehäusevorläufers, wobei zunächst ein weiterer Teilbereich des Pumpengehäuses aus dem weiteren Material gebildet wird, dann ein erster Teilbereich des Pumpengehäuses aus dem ersten Material und wobei ein zweiter weiterer Teilbereich des Pumpengehäuses aus dem weiteren Material gebildet wird;
d) Behandeln des Pumpengehäusevorläufers bei einer Temperatur von mindestens 300 °C.

Das Bereitstellen des ersten Materials in Schritt a) und des weiteren Materials in Schritt b) kann wie bereits bei dem zuvor beschriebenen Verfahren zur Herstellung eines Pumpengehäuses durchgeführt werden.

Das Bilden des Pumpengehäusevorläufers in Schritt c) kann auf jede beliebige Art und Weise unter Beachten der angegebenen Abfolge durchgeführt werden, die der Fachmann für den Zweck der Bildung eines ersten Teilbereiches und eines weiteren Teilbereiches auswählen würde. Details zu einzelnen Unterschritten wurden bereits in dem zuvor beschriebenen Verfahren angegeben.

Die Pumpvorrichtung ist erhältlich durch Einsetzen eines Impellers in ein Pumpengehäuse, Anordnen von Elektromagneten mit Spulen um das Pumpengehäuse, Herstellen eines Stromkreises unter Einbeziehen einer Steuervorrichtung und einer Stromquelle, z.B. einer Batterie. Bevorzugt wird die Pumpvorrichtung von einem Bauteilgehäuse umgeben und die weiteren Teilbereiche des Pumpengehäuses mit dem Bauteilgehäuse stoffschlüssig verbunden. Dies kann zum Beispiel durch eine Lötverbindung entlang der Berührungspunkt von Pumpengehäuses und Bauteilgehäuse durchgeführt werden.

Das Herstellen eines Pumpengehäuses kann einen Schritt c. beinhalten, einen Formgebungsprozess, bevorzugt ausgewählt aus der Gruppe bestehend aus einem lithographischen Prozess, einem Spritzgießen, einem Zerspanen, einem Extrudieren oder einer Kombination von mindestens zwei hiervon. Details zu den verschiedenen Formgebungsprozessen wurden für das zuvor beschriebene Herstellverfahren eines Pumpengehäusevorläufers beschrieben. Die Eigenschaften und Parameter die dort erwähnt wurden, gelten auch für diesen Herstellungsprozess.

Bei dem Bilden des Pumpengehäusevorläufers in Schritt c. kann ein erstes Material, zur Bildung eines ersten Teilbereiches mit einem weiteren Material, zur Bildung des weiteren Teilbereiches in Kontakt gebracht werden. Das Inkontaktbringen findet bevorzugt in Form eines Spritzgießens statt, bei dem nacheinander zunächst das weitere Material in eine Form aus Metall gespritzt wird und anschließend das ersten Material. Die Zusammensetzung des ersten und des weiteren Materials entsprechen bevorzugt den Zusammensetzungen des ersten oder weiteren Teilbereiches, wie sie oben im Zusammenhang mit dem ersten Gegenstand, der erfindungsgemäßen Pumpvorrichtung, beschrieben wurden. Bevorzugt weist der Pumpengehäusevorläufer nach dem Inkontaktbringen bereits die Form des Pumpengehäuses auf. Bevorzugt bilden die beiden Materialien eine kontinuierliche Form. Weiterhin wird erneut ein weiterer Teil des weiteren Materials mit dem ersten Material in Kontakt gebracht. Auf diese Weise entsteht ein Pumpengehäusevorläufer, der im Verlauf seiner röhrenförmigen Form zunächst einen ersten weiteren Teilbereich des weiteren Materials aufweist, benachbart von einem ersten Teilbereich aus dem ersten Material. Dieser erste Teilbereich ist wiederum benachbart von einem zweiten weiteren Teilbereich aus weiterem Material. Die Ausdehnungen und Zusammensetzungen der Teilbereiche entsprechen denen zu der erfindungsgemäßen Pumpvorrichtung angegebenen. Das Inkontaktbringen kann noch einen oder mehrere weitere Schritte beinhalten. So kann ein drittes Material, das bevorzugt eine Zusammensetzung wie der dritte Teilbereich der zuvor beschriebenen erfindungsgemäßen Pumpvorrichtung, zwischen dem ersten Material und dem weiteren Material in den Pumpengehäusevorläufer eingebracht werden.

Das Behandeln des Pumpengehäusevorläufers in Schritt d. kann auf jede beliebige Art und Weise erfolgen, die der Fachmann für den Zweck der Erwärmung des Pumpengehäusevorläufers auf mindestens 300 °C auswählen würde. Bevorzugt findet mindestens ein Teil des Behandelns des Pumpengehäusevorläufers bei einer Temperatur in einem Bereich von 300 bis 2500 °C, oder in einem Bereich von 500 bis 2000 °C, oder in einem Bereich von 700 bis 1800 °C statt. Bei der Behandlung des Pumpengehäusevorläufers bei erhöhter Temperatur entweicht bevorzugt mindestens ein Teil des Bindemittels. Es sind verschiedene Temperaturprofile beim Behandeln in Schritt d. des Pumpengehäusevorläufers aus Schritt c. möglich. Das Behandeln des Pumpengehäusevorläufers kann beispielsweise in einer oxidativen Atmosphäre, einer reduktiven Atmosphäre oder unter einer Schutzatmosphäre erfolgen. Eine oxidative Atmosphäre kann beispielsweise Sauerstoff enthalten, wie Luft oder ein Sauerstoff/Luft Gemisch. Eine reduktive Atmosphäre kann beispielsweise Wasserstoff enthalten. Eine Schutzatmosphäre beinhaltet bevorzugt weder Sauerstoff noch Wasserstoff. Beispiele für Schutzatmosphären sind Stickstoff, Helium, Argon, Krypton oder deren Gemische. Es können auch bevorzugt nacheinander Kombinationen von unterschiedlichen Atmosphären für verschiedene Zeiträume gewählt werden.

Das Behandeln des Pumpengehäusevorläufers kann entweder in einem Schritt erfolgen oder bevorzugt in mehr als einem Schritt. Bevorzugt wird der Pumpengehäusevorläufer in einem ersten Teilschritt des Schrittes d. auf eine Temperatur in einem Bereich von 301 bis 600 °C, oder bevorzugt in einem Bereich von 350 bis 550 °C, oder bevorzugt in einem Bereich von 400 bis 500 °C behandelt. Dieser erste Teilschritt des Behandlungsschrittes d. kann über einen Zeitraum in einem Bereich von 0,1 bis 100 h, bevorzugt in einem Bereich von 1 bis 50 h, oder bevorzugt in einem Bereich von 2 bis 20 h erfolgen. Dieser Teilschritt kann entweder durch Einbringen des Pumpengehäusevorläufers aus Schritt c. in eine vorgeheizte Atmosphäre erfolgen oder durch langsames schrittweises oder stetig erhöhtes Erhitzen des Pumpengehäusevorläufers. Bevorzugt wird das Behandeln in dem ersten Teilschritt des Schritt d. des Pumpengehäusevorläufers in einem Schritt auf eine Temperatur in einem Bereich von 301 bis 600 °C vorgenommen.

In einem zweiten Teilschritt des Behandelns aus Schritt d., der sich bevorzugt an den ersten Teilschritt anschließt, wird der Pumpengehäusevorläufer bevorzugt auf eine Temperatur in einem Bereich von 800 bis 2500 °C, oder bevorzugt in einem Bereich von 1000 bis 2000 °C, oder bevorzugt in einem Bereich von 1100 bis 1800 °C erhitzt. Auch dieser Teilschritt kann entweder durch Einbringen des Pumpengehäusevorläufers aus dem ersten Teilschritt des Schritt d. in eine vorgeheizte Atmosphäre erfolgen oder durch langsames schrittweises oder stetig erhöhtes Erhitzen des Pumpengehäusevorläufers. Bevorzugt wird das Behandeln in dem zweiten Teilschritt des Schritt d. des Pumpengehäusevorläufers in einem Schritt auf eine Temperatur in einem Bereich von 800 bis 2500 °C vorgenommen Die Behandlung des Pumpengehäusevorläufers in dem zweiten Teilschritt wird über einen Zeitraum in einem Bereich von 0,1 bis 20 h, bevorzugt in einem Bereich von 1 bis 12 h, oder bevorzugt in einem Bereich von 2 bis 5 h vorgenommen. Die Form des Pumpengehäuses nach dem Herstellungsprozess ist wie zuvor ausgeführt bevorzugt kontinuierlich.

Ein weiterer Gegenstand der vorliegenden Erfindung ist ein Gehäuse, das einen Innenbereich zumindest zu einem Teil umgibt, mit einem ersten Ende und einem zweiten Ende,
wobei das Gehäuse mindestens einen ersten Teilbereich und mindestens einen weiteren Teilbereich aufweist;
wobei der erste Teilbereich zu mindestens 61 Gew.-%, bezogen auf die Gesamtmasse des ersten Teilbereiches, eine Keramik beinhaltet,
wobei der weitere Teilbereich einen Metallgehalt in einem Bereich von 40 bis 90 Gew.-%, oder bevorzugt in einem Bereich von 45 bis 85 Gew.-%, oder bevorzugt in einem Bereich von 50 bis 80 Gew.-%, bezogen auf die Gesamtmasse des weiteren Teilbereiches bezogen auf die Gesamtmasse des weiteren Teilbereiches, beinhaltet,
wobei mindestens ein Teil des ersten Teilbereiches und mindestens ein Teil des weiteren Teilbereiches miteinander verbunden sind.

Das Gehäuse entspricht in seiner Form, seiner Zusammensetzung und seiner sonstigen Ausgestaltung dem Pumpengehäuse, das zuvor im Zusammenhang mit der erfindungsgemäßen Pumpvorrichtung beschrieben wurde.

In einer bevorzugten Ausgestaltung des Gehäuses ist in dem Gehäuse zumindest in einem Teil des Gehäuses ein verschiebbares Element vorgesehen ist. Weitere bevorzugte Ausführungsformen entsprechen den zuvor beschriebenen Ausführungsformen der erfindungsgemäßen Pumpvorrichtung.

Das verschiebbare Element kann ausgewählt sein aus der Gruppe bestehend aus einer Kugel, einem Zylinder, einer Luftblase oder einer Kombination aus mindestens zwei hiervon. Das verschiebbare Element weist bevorzugt eine Form auf, die dem Durchmesser des Pumpengehäuses entspricht. Das Material des verschiebbaren Elements kann jedes sein, das der Fachmann hierfür verwenden würde. Bevorzugt beinhaltet das verschiebbare Element ein Metall, ein Polymer, eine Keramik oder eine Mischung hieraus. Das Metall oder das Polymer kann ausgewählt sein aus einem Metall, einem Polymer oder einer Keramik wie sie für den ersten Teilbereich für das Pumpengehäuse beschrieben wurde. Das verschiebbare Element kann dazu dienen beispielsweise durch eine Änderung des Fluidstroms in dem Gehäuse in seiner Position in dem Gehäuse verschoben zu werden. Bei Änderung der Position des verschiebbaren Elements kann ein Stromfluss in einer Spule ausgelöst und mittels einer Stromflussmessung aufgezeichnet werden..

Ein weiterer Gegenstand der vorliegenden Erfindung ist eine Pumpvorrichtung beinhaltend mindestens ein zuvor beschriebenes Gehäuse.

### Messmethoden

### 1. Bestimmung der Härte nach Vickers (HV):

Die Prüfkräfte und Materialien wurden gemäß der Norm nach DIN EN ISO 6507-März 2006 bestimmt. Es wurden folgende Prüfkräfte und Einwirkdauern verwendet: 1 kg, 15 Sekunden. Die Prüftemperatur betrug 23 °C ± 1 °C

2. Bestimmung der magnetischen Permeabilität: Die magnetische Permeabilität wurde gemäß der Norm ASTM 773-01:2009 bestimmt.

### 3. Bestimmung der Biokompatibilität:

Die Biokompatibilität wird gemäß der Norm nach ISO 10993-4:2002 bestimmt.

### 4. Bestimmung der Hermetischen Verbindung:

Lecktests werden mit Heliumlecktestern und/oder Massenspektrometern durchgeführt werden. Ein Standardmessverfahrenist im Standard Mil-STD-883G Method 1014 spezifiziert. Die maximal zulässige Helium-Leckrate wird dabei abhängig vom internen Volumen der zu prüfenden Vorrichtung festgelegt. Nach den in MIL-STD-883G, Method 1014, in Absatz 3.1 spezifizierten Methoden, und unter Berücksichtigung der in der Anwendung der vorliegenden Erfindung vorkommenden Volumina und Kavitäten der zu prüfenden Vorrichtungen, beträgt die maximal zulässige Helium-Leckrate für die erfindungsgemäßen Pumpengehäuse 10⁻⁷ atm*cm³/sec oder weniger. Das bedeutet, dass die zu prüfende Vorrichtung (beispielsweise das Bauteilgehäuse und/oder die Pumpvorrichtung oder das Bauteilgehäuse mit dem verbundenen Pumpengehäuse) eine Helium-Leckrate von weniger als 1 x 10⁻⁷ atm*cm³/sec oder weniger aufweist. Für Vergleichszwecke können die genannten Helium-Leckraten auch in die äquivalente Standard-Luft-Leckrate konvertiert werden. Die Definition für die äquivalente Standard-Luft-Leckrate (Equivalent Standard Air Leak Rate) und die Umrechnung sind im Standard ISO 3530 angegeben.

### 5. Bestimmung der Rauheit:

Die Rauheit wird nach der Norm EN ISO 25178 bestimmt.

### Beispiele

### Beispiel 1 für erstes Material:

Das erste Material enthält 20 Teile Aluminiumoxid (Al₂O₃) erhältlich bei der Firma CeramTech GmbH, mit einer Korngröße von D₉₀ = 2 µm und einen Teil des Bindemittels METAWAX P-50 erhältlich bei der Firma Zschimmer & Schwarz GmbH & Co.KG.

### Beispiel 2 für weiteres Material:

Das weitere Material enthält eine Mischung aus 57 Gew.-% eines Platinpulvers der Firma Heraeus Precious Metals GmbH & Co.KG mit einer Korngröße D₅₀ = 50 m, und 38 Gew.-% Aluminiumoxid (Al₂O₃) der Firma CeramTech GmbH mit einer Korngröße von D₉₀ = 2 µm sowie 5 Gew.-% eines Bindemittels METAWAX P-50 erhältlich bei der Firma Zschimmer & Schwarz GmbH & Co.KG.

### Beispiel 3 für ersten Teilbereich:

Der erste Teilbereich enthält zu 100 Gew.-% Aluminiumoxid (Al₂O₃) der Firma CeramTech GmbH

### Beispiel 4 für weiteren Teilbereich:

Der weitere Teilbereich enthält zu 60 Gew.-% Platin und zu 40 Gew.-% Aluminiumoxid (Al₂O₃) der Firma CeramTech GmbH.

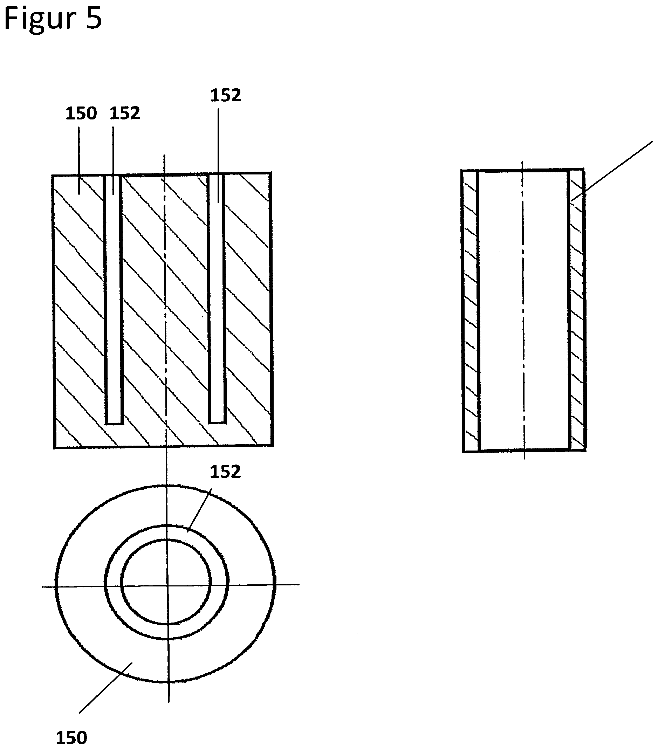

Das erste Material aus Beispiel 1 wird gemäß dem erfindungsgemäßen Verfahren für die Herstellung eines Pumpengehäuses zunächst in einem Behälter bereitgestellt. Das weitere Material aus Beispiel 2 wird ebenfalls in einem Behälter bereitgestellt. In abwechselnder Reihenfolge werden die Pulver des weiteren Materials und des ersten Materials in die Form, wie in Figur 5 gezeigt, gegeben und mit einem Stempel zusammengedrückt. Auf diese Weise wird ein Pumpengehäusevorläufer erhalten, das in einem Ofen zunächst bei einer Temperatur von 400 °C behandelt und anschließend bei einer Temperatur von 1700 °C gesintert wird, wobei ein Pumpengehäuse erhalten wird.

Im Folgenden wird in
- Figur 1: eine schematische Darstellung einer erfindungsgemäßen Pumpvorrichtung;
- Figur 2: ein Schema eines Verfahrens zur Herstellung eines erfindungsgemäßen Pumpengehäuses;
- Figur 3a-c: eine schematische Darstellung eines erfindungsgemäßen Pumpengehäuses mit einem ersten und einem weiteren Teilbereich direkt benachbart zueinander angeordnet;
- Figur 4a-c: eine schematische Darstellung eines erfindungsgemäßen Pumpengehäuses mit einem ersten und einem weiteren Teilbereich getrennt durch einen dritten Teilbereich angeordnet;
- Figur 5: ein Schema einer Pressvorrichtung zum Herstellen eines Pumpengehäusevorläufers;
gezeigt.

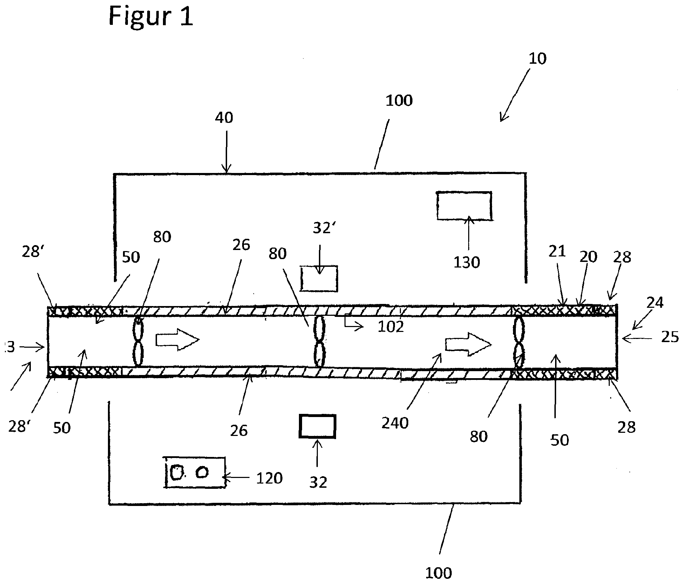

In Figur 1 ist schematisch eine Pumpvorrichtung 10 gezeigt, die ein Pumpgehäuse 20, in Form eines Rohres aufweist, sowie ein Bauteilgehäuse 40. Die Außenflächen 100 des Bauteilgehäuses 40 kommen insbesondere für eine implantierbare Pumpvorrichtung 10 mit dem Körper in Kontakt und sind daher bevorzugt biokompatibel ausgestaltet Das Pumpengehäuse 20 weist eine Wand 21 auf, die einen Innenbereich 50 umgibt. Die zum Innenbereich 50 weisende Fläche des Pumpengehäuses 20 wird als zugewandte Oberfläche 102 bezeichnet. Die zugewandte Oberfläche 102 kommt mit dem Fluid in Kontakt und ist daher insbesondere für eine implantierbare Pumpvorrichtung 10 bevorzugt biokompatibel ausgestaltet. In dem Innenbereich 50 des Pumpengehäuses 20 befindet sich mindestens ein Impeller 80, in diesem Fall befinden sich zwei Impeller 80 in dem Pumpengehäuse 20. Das Pumpengehäuse 20 weist einen ersten Teilbereich 26 in der Mitte der Wand 21 auf. An dem ersten Ende 22, das gleichzeitig den Einlass 22 durch die Öffnung 23 definiert, weist die Wand 21 bzw. das Pumpengehäuse 20 einen ersten weiteren Teilbereich 28 auf. Auf der gegenüberliegenden Seite des Pumpengehäuses 20 befindet sich das weitere Ende 24, in Form des Auslasses 24, beinhaltend die weitere Öffnung 25. Mittels des Impellers 80 kann ein Fluid in Pumprichtung 240 von dem Einlass 22 zum Auslass 24 gepumpt werden. Zwischen dem Bauteilgehäuse 40 und dem Pumpgehäuse befinden sich weitere Bauteile, wie eine Batterie 120 sowie eine Steuereinheit 130. Weiterhin befinden sich zwei Spulen 32 und 32' in dem Bauteilgehäuse 40.

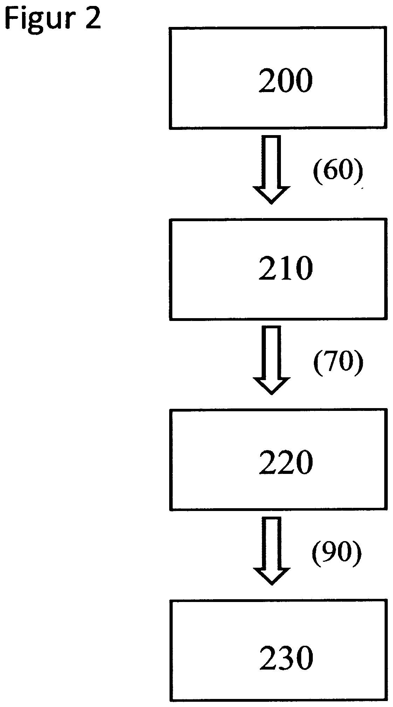

In Figur 2 ist schematisch der Ablauf des Verfahrens zur Herstellung eines Pumpengehäuses gezeigt. In dem Schritt a. bzw. a) 200 wird ein erstes Material 60 bereitgestellt. Das erste Material 60 ist beispielsweise eine Mischung aus mindestens zwei Pulvern. Das erste Material enthält die Zusammensetzung aus Beispiel 1, nämlich 20 Teile Aluminiumoxid (Al₂O₃) mit einer Korngröße von D₉₀ = 2 µm und ein Teil eines Bindemittels, in diesem Fall METAWAX P-50 erhältlich bei der Firma Zschimmer & Schwarz GmbH & Co. KG.

Das weitere Material 70 wird in Form einer Mischung, entsprechend Beispiel 2, aus 57 Gew.-% eines Platinpulvers mit einer Korngröße D₅₀ = 50 µm, und 38 Gew.-% Aluminiumoxid (Al₂O₃) mit einer Korngröße von D₉₀ = 2 µm sowie 5 Gew.-% eines Bindemittels, in diesem Fall METAWAX P-50 erhältlich bei der Firma Zschimmer & Schwarz GmbH & Co. KG in einem Schritt b. bzw. b) 210 ebenfalls bevorzugt in einem Behälter bereitgestellt. Der Behälter kann ein Metallbehälter mit einem Siebausgang sein. Bevorzugt weisen die Pulverkörner eine runde bis ovale Ausdehnung auf. Die Korngrößenangabe D₅₀ bedeutet, dass nicht mehr als 50 % der Teilchen größer sind als der angegebene Durchmesser. Die Korngrößenangabe D₉₀ bedeutet, dass nicht mehr als 90 % der Teilchen größer sind als der angegebene Durchmesser. Die Korngröße kann mit verschiedenen Methoden bestimmt werden. Bevorzugt wird die Korngröße mit Hilfe von Laserbeugung, Lichtmikroskopie, optische Einzelpartikelzählung oder einer Kombination mindestens zwei hiervon bestimmt. Weiterhin bevorzugt wird die Bestimmung der Korngröße so wie der Korngrößenverteilung anhand von optischer Einzelauswertung von Aufnahmen mittels Transmissions-Elektronen-Mikroskopie (TEM) vorgenommen. Außerdem kann die Korngröße dem Produktdatenblatt entnommen werden, das beim Rohstofflieferanten verfügbar und oftmals einer Lieferung beigepackt ist.

In einem Schritt c bzw. c) 220 wird aus dem ersten Material 60 und dem weiteren Material 70 ein Pumpengehäusevorläufer 90 gebildet.

Die Schritte c. bzw. c) 200 sind zwei Alternativen nach denen bei der Bildung des Pumpengehäusevorläufers 90 verfahren werden kann. In der ersten Alternative des Schrittes c. wird zunächst ein weiterer Teilbereich 28 durch das weitere Material 70 gebildet. Hierbei wird das weitere Material 70 mit Hilfe einer Teflonrakel mit den Dimensionen 10 mm * 4 mm * 2 mm und einer Rakelhärte von 50 shore in eine Form 150 aus einer Aluminiumoxidkeramik, wie in Figur 5 schematisch gezeigt, gedruckt. Das weitere Material 70 wird bis zu einer Füllhöhe von 5 mm in die Form 150 eingefüllt. Anschließend wird das erste Material 60 in die Form 150 aus Figur 5 gedrückt. Das erste Material 60 wird bis zu einer Füllhöhe von 25 mm eingefüllt. Mit einem Stempel aus Edelstahl wird das erste und das weitere Material 70 unter einem Druck von einem Gewicht von 5 Kg zusammengedrückt. In dem so geformten Pumpengehäusevorläufer 90 liegt folglich der erste Teilbereich 26 des Pumpengehäuses 20 aus dem ersten Material 60 neben dem weiteren Teilbereich 28 des Pumpengehäuses 20 vor.

In der Alternative des Schrittes c) wird genauso vorgegangen wie zuvor für den Schritt c. beschrieben, mit dem Unterschied, dass das erste Material 60 nur bis zu einer Füllhöhe von 25 mm eingefüllt wird. Anschließend wird ein zweiter weiterer Teilbereich des Pumpenrohrvorläufers 90 gebildet, indem erneut ein weiteres Material 70, wie oben beschrieben bis zu einer Füllhöhe von 30 mm in die Form 150 eingefüllt wird. Auf diese Weise wird ein Pumpengehäusevorläufer 90 erhalten, der eine Abfolge von einem ersten weiteren Teilbereich 28, 28' neben einem ersten Teilbereich 26, 26' und einem zweiten weiteren Teilbereich 28, 28' vorsieht. Die Dicke des ersten weiteren Teilbereiches 28', 28, des ersten Teilbereiches 26, 26' und des zweiten weiteren Teilbereiches 28, 28' sind identisch. Durch die Wahl der Form der Form 150 in die die Materialien gegeben werden kann erreicht werden, dass die Wanddicken mindestens in Teilen der Teilbereiche 26, 28, 30 unterschiedlich ist.

Anschließend wird der Pumpengehäusevorläufer 90 bei einer Temperatur von 400 °C an Luft behandelt. Diese Behandlung findet in einem Heizofen der Firma Heraeus Holding GmbH für einen Zeitraum von 60 min statt. Direkt im Abschluss an diesen Behandlungsschritt wird der Pumpengehäusevorläufers 90 bei einer Temperatur von 1700 °C in dem gleichen Ofen für 180 min behandelt, wobei die Teilbereiche 26, 28 zusammen sintern und ein Pumpengehäuse entsteht. Es entsteht ein Pumpengehäuse in Form eines runden Rohres mit einem Innendurchmesser von 9 mm. Die Wandstärke in allen Bereichen beträgt 1 mm.

Figuren 3a bis 3c zeigen drei verschiedene Möglichkeiten der Anordnung von mehreren Teilbereichen in einem erfindungsgemäßen Pumpengehäuse 20. Das Pumpengehäuse 20 beinhaltet in seiner Wand 21 eine erste Öffnung 23 am Einlass 22 und eine weitere Öffnung 25 am Auslass 24. In der Figur 3a ist ein erster Teilbereich 26, in der Mitte der Wand 21 des Pumpengehäuses 20 angeordnet. Rechts und links davon befinden sich je ein weiterer Teilbereich 28, und 28'. Die beiden weiteren Teilbereiche 28 und 28' sind parallel zu einander und zu dem ersten Teilbereich 26 angeordnet.

In Figur 3b ist ebenfalls ein Pumpengehäuse 20 mit Wand 21 sowie einem ersten Teilbereich 26 in der Mitte der Wand 21 gezeigt. Auch hier schließen sich an dem Einlass 22 und Auslass 24 jeweils ein weiterer Teilbereich 28 und 28'an. Im Unterschied zu dem Pumpengehäuse 20 aus Figur 3a sind die weiteren Teilbereiche 28 und 28' nicht in dem gesamten Umfang der Pumpenvorrichtung parallel zu dem ersten Teilbereich 26 angeordnet sondern weisen über den Umfang des Pumpengehäuses 20 unterschiedlich breite Abschnitte auf. Dies führt zu einem schrägen Profil des Übergangs vom ersten 26 zu den weiteren Teilbereichen 28, 28'.

Auch in Figur 3c ist ein nicht paralleler Übergang von den weiteren Teilbereichen 28, 28' zu dem ersten Teilbereich 26 gezeigt. Hier weist der Übergang einen Versatz auf. Ansonsten ist das Pumpengehäuse 20 aus Figur 3c, wie das aus Figur 3a aufgebaut.

Die Figuren 4a, 4b und 4c zeigen jeweils einen ähnlichen Aufbau wie die Figuren 3a, 3b und 3c eines Pumpengehäuses 20 auf, jedoch sind hier zwischen jeweils einem ersten Teilbereich 26 und den weiteren Teilbereich 28 und 28' ein dritter Teilbereich 30 und 30' angeordnet. Ansonsten entsprechen die Aufbauten der Pumpengehäuse aus Figur 4a dem der Figur 3a, die der Figur 4b dem aus 3b und der Aufbau des Pumpengehäuses aus Figur 4c dem des Pumpengehäuses aus Figur 3c.

In Figur 5 ist eine Form 150 gezeigt, die aus einem massiven Material, wie einer Keramik oder einem Edelstahl gefertigt ist. In der Form 150 ist eine runde Aussparung 152 ein gefräst die sich an einer Seite der Form 150 nach außen öffnet. Durch die Öffnung kann Material in die Form 150 eingefüllt werden, das beispielsweise pastenförmig oder pulverförmig ist und mit dem Stempel 160, der genau in die Öffnung der Form 150 passt zusammengepresst werden. Auf diese Weise kann ein Rohling oder ein Vorläufer für ein Pumpengehäuse 20 in Form eines Rohres geschaffen werden, wie dies bereits für das Verfahren in Figur 2 beschrieben wurde.

**Bezugszeichenliste**

| | | | |
|---|---|---|---|
| 10 | Pumpvorrichtung | 240 | Pumprichtung |
| 20 | Gehäuse, Pumpengehäuse | | |
| 21 | Wand | | |
| 22 | erstes Ende / Einlass | | |
| 23 | erste Öffnung | | |
| 24 | weiteres Ende /Auslass | | |
| 25 | weitere Öffnung | | |
| 26 | erste Teilbereich | | |
| 28, 28' | weiterer Teilbereich | | |
| 30, 30' | dritter Teilbereich | | |
| 40 | Bauteilgehäuse | | |
| 50 | Innenbereich | | |
| 60 | erstes Material | | |
| 70 | weiteres Material | | |
| 80 | Impeller | | |
| 90 | Vorläufer/Pumpengehäusevorläufer | | |
| 100 | Außenfläche | | |
| 102 | zugewandte Oberfläche | | |
| 110 | elektrisches Bauteil | | |
| 120 | Batterie | | |
| 130 | Steuereinheit | | |
| 150 | Form | | |
| 152 | Aussparung | | |
| 160 | Stempel | | |
| 200 | Schritt a. / Schritt a) | | |
| 210 | Schritt b. / Schritt b) | | |
| 220 | Schritt c. / Schritt c) | | |
| 230 | Schritt d. / Schritt d) | | |

## Patentansprüche

1. Eine Pumpvorrichtung (10), beinhaltend:
i. einen Impeller (80);
ii. ein Pumpengehäuse (20), das einen Innenbereich (50) zumindest zu einem Teil umgibt, mit einem Einlass (22) und einem Auslass (24),
wobei der Impeller (80) im Innenbereich (50) des Pumpengehäuses (20) vorgesehen ist;
wobei das Pumpengehäuse (20) mindestens einen ersten Teilbereich (26) und mindestens einen weiteren Teilbereich (28) beinhaltet;
wobei der erste Teilbereich (26) zu mindestens 61 Gew.-%, bezogen auf die Gesamtmasse des ersten Teilbereiches (26), eine Keramik beinhaltet,
wobei der weitere Teilbereich (28) einen Metallgehalt in einem Bereich von 40 bis 90 Gew.-%, bezogen auf die Gesamtmasse des weiteren Teilbereiches (28), beinhaltet,
wobei mindestens ein Teil des ersten Teilbereiches (26) und mindestens ein Teil des weiteren Teilbereiches (28) miteinander verbunden sind.

2. Die Pumpvorrichtung (10) nach Anspruch 1, wobei die Keramik des mindestens einen ersten Teilbereiches (26) ausgewählt ist aus der Gruppe bestehend aus Aluminiumoxid (Al₂O₃), Zirkoniumdioxid (ZrO₂), einem ein Aluminiumoxid enthaltendes Zirkoniumoxid (ATZ), einem ein Zirkoniumoxid enthaltendes Aluminiumoxid (ZTA), einem ein Yttrium enthaltendes Zirkoniumoxid (Y-TZP), Aluminiumnitrid (AIN), Titannitrid (TiN), Magnesiumoxid (MgO), einer Piezokeramik, Barium(Zr, Ti)oxid, Barium(Ce, Ti)oxid und Natrium-Kalium-Niobat oder einer Mischung aus mindestens zwei hiervon.

3. Die Pumpvorrichtung (10) nach einem der vorhergehenden Ansprüche, wobei das Metall des mindestens einen weiteren Teilbereiches (28) ausgewählt ist aus der Gruppe bestehend Platin (Pt), Eisen (Fe), Edelstahl (AISI 304, AISI 316 L), Iridium (Ir), Niob (Nb), Molybdän (Mo), Wolfram (W), Titan (Ti), Kobalt (Co), Chrom (Cr), eine Kobalt-Chrom-Legierung, Tantal (Ta) und Zirkonium (Zr) oder einer Mischung aus mindestens zwei hiervon.

4. Die Pumpvorrichtung (10) nach einem der vorhergehenden Ansprüche, wobei der mindestens eine erste Teilbereich (26) weniger als 10 Gew.-%, bezogen auf die Gesamtmasse des ersten Teilbereiches (26), an Metall beinhaltet.

5. Die Pumpvorrichtung (10) nach einem der vorhergehenden Ansprüche, wobei der mindestens eine erste Teilbereich (26) und der mindestens eine weitere Teilbereich (28) durch mindestens einen dritten Teilbereich (30) miteinander verbunden sind.

6. Die Pumpvorrichtung (10) nach dem vorhergehenden Anspruch, wobei der mindestens eine dritte Teilbereich (30) einen Metallgehalt zwischen dem Metallgehalt des ersten Teilbereiches (26) und dem Metallgehalt des weiteren Teilbereiches (28) aufweist.

7. Die Pumpvorrichtung (10) nach einem der vorhergehenden Ansprüche, wobei die Pumpvorrichtung zumindest zu einem Teil von einem Bauteilgehäuse (40) umgeben ist, wobei mindestens ein Teil des mindestens einen weiteren Teilbereiches (28) der Pumpvorrichtung (10) mit dem Bauteilgehäuse (40) verbunden ist.

8. Die Pumpvorrichtung (10) nach dem vorhergehenden Anspruch, wobei das Bauteilgehäuse (40) mindestens 30 Gew.-%, bezogen auf die Gesamtmasse des Bauteilgehäuses (40) Titan beinhaltet.

9. Die Pumpvorrichtung (10) nach einem der vorhergehenden Ansprüche, wobei die Wand (21) des Pumpengehäuses (20) eine magnetische Permeabilität von weniger als 2 µ aufweist.

10. Die Pumpvorrichtung (10) nach einem der vorhergehenden Ansprüche, wobei die Oberfläche (102) des mindestens einen ersten Teilbereiches (26), die dem Innenbereich (50) des Pumpengehäuses (20) zugewandt ist, eine Härte nach Vickers von mindestens 330 HV, bevorzugt mindestens 350 HV, bevorzugt mindestens 370 HV aufweist.

11. Ein Gehäuse (20), das einen Innenbereich (50) zumindest zu einem Teil umgibt, mit einem ersten Ende (22) und einem zweiten Ende (24),
wobei das Gehäuse (20) mindestens einen ersten Teilbereich (26) und mindestens einen weiteren Teilbereich (28) aufweist;
wobei der erste Teilbereich (26) zu mindestens 61 Gew.-%, bezogen auf die Gesamtmasse des ersten Teilbereiches (26), eine Keramik beinhaltet,
wobei der weitere Teilbereich (28) einen Metallgehalt in einem Bereich von 40 bis 90 Gew.-%, bezogen auf die Gesamtmasse des weiteren Teilbereiches (28), beinhaltet,
wobei mindestens ein Teil des ersten Teilbereiches (26) und mindestens ein Teil des weiteren Teilbereiches (28) miteinander verbunden sind.

12. Eine Pumpvorrichtung (10) beinhaltend mindestens ein Gehäuse (20) nach Anspruch 11

## Claims

1. A pumping device (10), including:
i. an impeller (80);
ii. a pump housing (20) at least partially surrounding an inner area (50), with an inlet (22) and an outlet (24),
wherein the impeller (80) is provided in the inner region (50) of the pump housing (20); wherein the pump housing (20) comprises at least one first partial area (26) and at least one further partial area (28);
wherein the first partial region (26) contains at least 61% by weight, based on the total mass of the first partial region (26), of a ceramic,
wherein the further subregion (28) contains a metal content in a range from 40 to 90% by weight, based on the total mass of the further subregion (28),
wherein at least a part of the first partial region (26) and at least a part of the further partial region (28) are connected to each other.

2. The pumping device (10) according to claim 1, wherein the ceramic of the at least one first partial region (26) is selected from the group consisting of alumina (Al₂O₃), zirconia (ZrO₂), a zirconia containing an alumina (ATZ), an alumina (ZTA) containing a zirconia, a zirconia (Y-TZP) containing an yttrium, aluminum nitride (AIN), titanium nitride (TiN), magnesium oxide (MgO), a piezoceramic, barium (Zr, Ti) oxide, barium (Ce, Ti) oxide and sodium potassium niobate or a mixture of at least two of them.

3. The pumping device (10) according to one of the preceding claims, wherein the metal of the at least one further subregion (28) is selected from the group consisting of platinum (Pt), iron (Fe), stainless steel (AISI 304, AISI 316 L), iridium (Ir), niobium (Nb), molybdenum (Mo), tungsten (W), titanium (Ti), cobalt (Co), chromium (Cr), a cobalt-chromium alloy, tantalum (Ta) and zirconium (Zr) or a mixture of at least two thereof.

4. The pumping device (10) according to any of the foregoing claims, wherein the at least one first portion (26) comprises less than 10% by weight, based on the total mass of the first portion (26), of metal.

5. The pumping device (10) according to one of the preceding claims, wherein the at least one first partial region (26) and the at least one further partial region (28) are connected to each other by at least one third partial region (30).

6. The pumping device (10) according to the preceding claim, wherein the at least one third partial region (30) has a metal content between the metal content of the first partial region (26) and the metal content of the further partial region (28).

7. The pump device (10) according to one of the preceding claims, wherein the pump device is surrounded at least in part by a component housing (40), wherein at least a part of the at least one further partial area (28) of the pump device (10) is connected to the component housing (40).

8. The pumping device (10) according to the preceding claim, wherein the component housing (40) contains at least 30% by weight, based on the total mass of the component housing (40), of titanium.

9. The pump device (10) according to any of the foregoing claims, wherein the wall (21) of the pump housing (20) has a magnetic permeability of less than 2 µ.

10. The pump device (10) according to one of the preceding claims, wherein the surface (102) of the at least one first partial area (26) facing the inner area (50) of the pump housing (20) has a Vickers hardness of at least 330 HV, preferably at least 350 HV, preferably at least 370 HV.

11. A housing (20) at least partially surrounding an interior region (50), having a first end (22) and a second end (24),
wherein the housing (20) has at least one first partial region (26) and at least one further partial region (28);
wherein the first partial region (26) contains at least 61% by weight, based on the total mass of the first partial region (26), of a ceramic,
wherein the further subregion (28) contains a metal content in a range from 40 to 90% by weight, based on the total mass of the further subregion (28),
wherein at least a part of the first partial region (26) and at least a part of the further partial region (28) are connected to each other.

12. A pump device (10) comprising at least one housing (20) according to claim 11.

## Revendications

1. Un dispositif de pompage (10), comprenant :
i. une roue (80) ;
ii. un corps de pompe (20) entourant au moins partiellement une région intérieure (50), ayant une entrée (22) et une sortie (24),
dans laquelle la roue (80) est prévue dans la zone intérieure (50) du corps de pompe (20) ;
dans lequel le corps de pompe (20) comprend au moins une première zone partielle (26) et au moins une autre zone partielle (28) ;
dans laquelle la première région partielle (26) contient au moins 61 % en poids, par rapport à la masse totale de la première région partielle (26), d'une céramique,
dans laquelle l'autre sous-région (28) contient une teneur en métal comprise entre 40 et 90 % en poids, par rapport à la masse totale de l'autre sous-région (28),
dans laquelle au moins une partie de la première région partielle (26) et au moins une partie de l'autre région partielle (28) sont reliées entre elles.

2. Le dispositif de pompage (10) selon la revendication 1, dans lequel la céramique de la au moins une première région partielle (26) est choisie dans le groupe constitué par l'alumine (Al₂O₃), la zircone (ZrO₂), une zircone contenant une alumine (ATZ), une alumine (ZTA) contenant une zircone, une zircone (Y-TZP) contenant un yttrium, du nitrure d'aluminium (AIN), du nitrure de titane (TiN), de l'oxyde de magnésium (MgO), une piézocéramique, de l'oxyde de baryum (Zr, Ti), de l'oxyde de baryum (Ce, Ti) et du niobate de sodium et de potassium ou un mélange d'au moins deux d'entre eux.

3. Le dispositif de pompage (10) selon l'une des revendications précédentes, dans lequel le métal d'au moins une autre sous-région (28) est choisi dans le groupe constitué par le platine (Pt), le fer (Fe), l'acier inoxydable (AISI 304, AISI 316 L), iridium (Ir), niobium (Nb), molybdène (Mo), tungstène (W), titane (Ti), cobalt (Co), chrome (Cr), alliage cobalt-chrome, tantale (Ta) et zirconium (Zr) ou un mélange d'au moins deux de ces éléments.

4. Le dispositif de pompage (10) selon l'une des revendications précédentes, dans lequel la au moins une première partie (26) comprend moins de 10% en poids, par rapport à la masse totale de la première partie (26), de métal.

5. Le dispositif de pompage (10) selon l'une des revendications précédentes, dans lequel la au moins une première région partielle (26) et la au moins une autre région partielle (28) sont reliées entre elles par au moins une troisième région partielle (30).

6. Le dispositif de pompage (10) selon la revendication précédente, dans lequel la au moins une troisième région partielle (30) a une teneur en métal comprise entre la teneur en métal de la première région partielle (26) et la teneur en métal de l'autre région partielle (28).

7. Le dispositif de pompage (10) selon l'une des revendications précédentes, le dispositif de pompage étant entouré au moins en partie par un boîtier de composants (40), au moins une partie de la au moins une autre zone partielle (28) du dispositif de pompage (10) étant reliée au boîtier de composants (40).

8. Le dispositif de pompage (10) selon la revendication précédente, dans lequel le boîtier de composant (40) contient au moins 30% en poids, par rapport à la masse totale du boîtier de composant (40), de titane.

9. Le dispositif de pompe (10) selon l'une des revendications précédentes, dans lequel la paroi (21) du corps de pompe (20)a une perméabilité magnétique inférieure à 2 µ.

10. Le dispositif de pompe (10) selon l'une des revendications précédentes, dans lequel la surface (102) de la au moins une première zone partielle (26) tournée vers la zone intérieure (50) du corps de pompe (20) présente une dureté Vickers d'au moins 330 HV, de préférence d'au moins 350 HV, de préférence d'au moins 370 HV.

11. Un logement (20) entourant au moins partiellement une région intérieure (50), ayant une première extrémité (22) et une deuxième extrémité (24),
dans lequel le logement (20) comporte au moins une première région partielle (26) et au moins une autre région partielle (28) ;
dans laquelle la première région partielle (26) contient au moins 61 % en poids, par rapport à la masse totale de la première région partielle (26), d'une céramique,
dans laquelle l'autre sous-région (28) contient une teneur en métal comprise entre 40 et 90 % en poids, par rapport à la masse totale de l'autre sous-région (28),
dans laquelle au moins une partie de la première région partielle (26) et au moins une partie de l'autre région partielle (28) sont reliées entre elles.

12. Le dispositif de pompage (10) comprenant au moins un boîtier (20) selon la revendication 11.
